# EUROPEAN PATENT SPECIFICATION

(11) **EP 3 758 641 B1**
(45) Date of publication and mention of the grant of the patent: **14.01.2026**
(21) Application number: 19760402.8
(22) Date of filing: 28.02.2019
(51) Int. Cl.: A61B 34/20, A61B 34/30, A61B 34/10, A61B 1/307, G06T 7/70, A61B 90/50, A61B 90/00, A61B 17/22, A61B 1/00, A61B 17/00

(54) **SYSTEMS FOR MAPPING AND NAVIGATION**
SYSTEME ZUR KARTIERUNG UND NAVIGATION
SYSTÈMES DE CARTOGRAPHIE ET DE NAVIGATION

(30) Priority: 01.03.2018 US 201862637048 P
(43) Date of publication of application: 06.01.2021
(73) Proprietor: Auris Health, Inc., Santa Clara, CA 95054 (US)
(72) Inventor: HSU, Jason Joseph, Mountain View, California 94040 (US); SRAMEK, Christopher, K., Redwood City, California 94065 (US); SHEEHY, Alexander James, Redwood City, California 94065 (US)
(74) Representative: Carpmaels & Ransford LLP
(86) International application number: PCT/US2019/020120
(87) International publication number: WO 2019/169178

(56) References cited:
- US-A1- 2017 303 941
- US-A1- 2017 365 055
- US-B2- 7 907 991
- VEMURI, A. ET AL.: "Inter-Operative Biopsy Site Relocalization in Endoluminal Surgery", IEEE TRANSACTIONS ON BIOMEDICAL ENGINEERING, vol. 63, no. 9, December 2015 (2015-12-01), pages 1862 - 1873, XP011620573
- SHI, C. ET AL.: "Simultaneous Catheter and Environment Modeling for Trans-catheter Aortic Valve Implantation", IEEE /RSJ INTERNATIONAL CONFERENCE ON INTELLIGENT ROBOTS AND SYSTEMS, 14 September 2014 (2014-09-14), pages 2024 - 2029, XP032676438, DOI: 10.1109/IROS.2014.6942832
- KONEN, W. ET AL.: "The VN -Project: Endoscopic Image Processing for Neurosurgery", COMPUTER AIDED SURGERY, vol. 3, no. 3, 1998, pages 144 - 148, XP055635135

## Description

### TECHNICAL FIELD

This disclosure relates generally to mapping and/or navigation of an interior region of a body, and more particularly, to methods and systems for mapping and/or navigation of an interior region of a body with a robotically-enabled medical instrument.

### BACKGROUND

Medical procedures such as endoscopy (e.g., ureteroscopy) may involve accessing and visualizing an interior region of a patient's body (e.g., a kidney) for diagnostic and/or therapeutic purposes.

Ureteroscopy is a medical procedure commonly used for the treatment of kidney stones. During the procedure, a thin, flexible tubular tool or instrument, known as a ureteroscope, may be inserted into the urethra, through the bladder and ureter, and into the kidney.

In certain procedures, a robotically-enabled medical system may be used to control the insertion and/or manipulation of the instrument. The robotically-enabled medical system may include a robotic arm, or other instrument positioning device, having a manipulator assembly used to control the positioning of the instrument during the procedure.
US2017/0365055A1 discusses methods and apparatuses for navigation through tubular networks such as lung airways by providing estimation of location and orientation information of a medical instrument (e.g., an endoscope) within the tubular network. Various input data such as image data, EM data, and robot data are used by different algorithms to estimate the state of the medical instrument, and the state information is used to locate a specific site within a tubular network and/or to determine navigation information for what positions/orientations the medical instrument should travel through to arrive at the specific site. Probability distributions together with confidence values are generated corresponding to different algorithms are used to determine the medical instrument's estimated state.
Vemuri, A et al. "Inter-Operatrive Biopsy Site Relocalization in Endoluminal Surgery" IEEE Transaction on Biomedical Engineering, vol. 63, no. 9, December 2015, pages 1862-1873 discusses an approach to provide guided navigation and relocalization of the biopsy sites using an electromagnetic tracking system. This approach utilizes the integration of an electromagnetic sensor at the flexible endoscope tip, so that the endoscopic camera depth inside the oesophagus can be computed in real time, allowing to retrieve and display an image from a previous exploration at the same depth.
US2017/303941A1 discusses systems and methods for guided removal from an in vivo subject. The method comprises, guiding a flexible tube through an in vivo subject's ureter, wherein the flexible tube comprises at least a first passageway and a second passageway. Positioning a distal end of the first passageway adjacent to the object. Infusing saline solution through the second passageway while suction is off. Removing the object through the first passageway with at least a portion of the saline solution.
Chi, C. et al.: "Simultaneous Catheter and Environment Modeling for Transcatheter Aortic Valve Implantation", IEEE /RSJ International Conference on Intelligent Robots and Systems, 14 September 2014 (2014-09-14), pages 2024-2029, DOI: 10.1109/IROS.2014.6942832 discusses a vasculature reconstruction and catheter modeling scheme based on data fusion from intravascular ultrasound (IVUS) imaging, electromagnetic (EM) tracking and shape sensing for trans-femoral Transcatheter Aortic Valve Implantation (TAVI). The system is suitable for obtaining inner cross sectional images of the aorta with an IVUS probe, reconstructing its 3D virtual model using sensor fusion of the corresponding pose information of IVUS probe from an electromagnetic (EM) sensor, as well as reconstructing the catheter shape based on optical fibers with Fiber Bragg Grating (FBG) sensors.
US7907991B2 discusses systems and methods for marking a body cavity. The system includes means for inspecting a body cavity, and means for visibly marking the body cavity so as to convey visual information regarding the body cavity. The method includes inspecting a body cavity, and marking the body cavity with a marking material to provide a visual indication regarding the cavity. The marking material for marking a body cavity includes a radiopaque contrast agent that is viewable through fluoroscopy, and a colored dye that is viewable using an internal viewing device.
Konen, W et al. "The VN -Project: Endoscopic Image Processing for Neurosurgery", Computer Aided Surgery, vol. 3, no. 3, 1998, pages 144-148 discusses a navigation support system for endoscopic interventions that allows three-dimensional (3-D) information to be extracted from endoscopic video data and superimposed onto such live video sequences. The endoscope is coupled to a position measurement system and a video camera as components of a calibrated system.

### SUMMARY

The presently claimed invention provides a non-transitory computer readable storage medium according to claim 1 and a robotic surgical system according to claim 15. Further developments of the herein claimed invention are described in the dependent claims. Embodiments of the disclosure relate to systems and techniques for mapping and/or navigation of an interior region of a body with a robotically-enabled medical instrument. The systems and techniques may be configured to generate visual indicia indicative of historical (e.g., previous) positions of the instrument as the instrument is navigated through the interior region. The visual indicia may be derived from positional information received from a position sensor. The visual indicia may be superimposed on a reference image of the interior region. The visual indicia may form a map that can be representative of the previous positions of the instrument. The map can be used to visualize the anatomy of the interior region and can be used by a physician to navigate the interior region. In some implementations, the visual indicia comprise points or a trace indicative of the path of the instrument as it navigates through the interior region. In some implementations, the visual indicia can comprise a mesh structure representative of the anatomy of the interior region.

The methods and techniques may also be configured to receive image data from an image sensor positioned on the instrument. The image data can include still images or videos. The methods and techniques can link the image data with the positional data, such that an image captured at a particular location can be recalled and displayed to the user. In some implementations, images are recalled and displayed when a user selects a position within the interior region or when the instrument is positioned at a position for which there is a linked image. Further, the methods and techniques may be configured to permit the physician to tag certain features or locations of interest.

Accordingly, one aspect relates to a method for mapping an interior region of a body. The method includes: displaying a reference image of the interior region of the body; moving an instrument within the interior region of the body, the instrument comprising at least one position sensor; receiving positional information from the at least one position sensor, the positional information comprising a plurality of positional data sets, each positional data set indicative of a position of the instrument during the movement of the instrument; and superimposing visual indicia derived from at least a subset of the positional data sets on the reference image to characterize historical positions of the instrument during the movement within the interior region of the body.

In some embodiments, the method can include one or more of the following features in any combination: (a) wherein the reference image comprises an image captured during a retrograde pyelogram procedure; (b) wherein the reference image comprises a fluoroscopic or an ultrasonic image; (c) wherein the reference image is captured during a computed tomography (CT) or magnetic resonance imaging (MRI) procedure; (d) capturing the reference image intraoperatively; (e) wherein the reference image is captured preoperatively; (f) receiving instrument image data from an imaging device positioned on the instrument, the instrument image data comprising a plurality of images captured by the imaging device during the movement of the instrument, and for at least a subset of the plurality of images, linking each image of the subset with the positional data set indicative of a position at which the image was captured; (g) comprising storing the linked images for use during a future procedure; (h) receiving a user input of a position selection, and displaying a linked image corresponding to the user input; (i) determining, with the position sensor, a current position of the instrument, and displaying a linked image corresponding to the determined current position; (j) wherein the instrument image data is captured automatically; (k) wherein the instrument image data is captured upon receipt of a user command; (l) tagging a location or feature of interest within the interior region; (m) wherein tagging comprises receiving a user input; (n) wherein tagging comprises automatically detecting the location or feature of interest; (o) superimposing the tagged location or feature of interest on the reference image; (p) connecting the visual indicia to characterize the historical path of the movement of the instrument; (q) wherein the visual indicia comprise a mesh; (r) wherein the mesh is indicative of anatomy of the interior region; (s) wherein the mesh is derived from the subset of the positional data sets and image data received from an imaging device on the instrument; (t) wherein the subset of the positional data sets is superimposed on the reference image at a durational frequency; (u) wherein the subset of the positional data sets is superimposed on the reference image at a positional frequency; (v) displaying the visual indicia intraoperatively; (w) storing the visual indicia for use during a future medical procedure; (x) wherein the interior region of the body comprises a kidney, and wherein the method further comprises: moving the instrument into a calyx of the kidney; and tagging at least one of: an entrance to the calyx of the kidney, a pole of the kidney, a stone within the kidney, and an area of transitional cell cancer; and/or (y) adjusting a position determined by the position sensor to account for a physiological movement, and superimposing the adjusted position on the reference image.

In another aspect, a non-transitory computer readable storage medium having stored thereon instructions is disclosed. The instructions, when executed, can cause a processor of a device to at least: move an instrument within an interior region of a body; receive positional information from at least one position sensor of the instrument during the movement of the instrument, the positional information comprising a plurality of positional data sets, each positional data set indicative of a position of the instrument; display an image of the interior region of the body; and superimpose visual indicia derived from at least a subset of the positional data sets on the image to characterize historical positions of the instrument during the movement of the instrument within the interior region of the body.

In some embodiments, the non-transitory computer readable storage medium can include one or more of the following features in any combination: (a) wherein the reference image comprises an image captured during a retrograde pyelogram procedure; (b) wherein the reference image comprises a fluoroscopic or an ultrasonic image; (c) wherein the reference image is captured during a computed tomography (CT) or magnetic resonance imaging (MRI) procedure.; (d) wherein the instructions, when executed, further cause the processor to capture the reference image intraoperatively; (e) wherein the reference image is captured preoperatively; (f) wherein the instructions, when executed, further cause the processor to: receive instrument image data from an imaging device positioned on the instrument, the instrument image data comprising a plurality of images captured by the imaging device during the movement of the instrument; and for at least a subset of the plurality of images, link each image of the subset with the positional data set indicative of a position at which the image was captured; (g) wherein the instructions, when executed, further cause the processor to store the linked images for use during a future procedure; (h) wherein the instructions, when executed, further cause the processor to: receive a user input of a position selection; and display a linked image corresponding to the user input; (i) wherein the instructions, when executed, further cause the processor to: determine, with the position sensor, a current position of the instrument; and display a linked image corresponding to the determined current position; (j) wherein the instrument image data is captured automatically; (k) wherein the instrument image data is captured upon receipt of a user command; (l) wherein the instructions, when executed, further cause the processor to tag a location or feature of interest within the interior region; (m) wherein tagging comprises receiving a user input; (n) wherein tagging comprises automatically detecting the location or feature of interest; (o) wherein the instructions, when executed, further cause the processor to superimpose the tagged location or feature of interest on the reference image; (p) wherein the instructions, when executed, further cause the processor to connect the visual indicia to characterize the historical path of the movement of the instrument; (q) wherein the visual indicia comprise a mesh; (r) wherein the mesh is indicative of anatomy of the interior region; (s) wherein the mesh is derived from the subset of the positional data sets and image data received from an imaging device on the instrument; (t) wherein the subset of the positional data sets is superimposed on the reference image at a durational frequency; (u) wherein the subset of the positional data sets is superimposed on the reference image at a positional frequency; (v) wherein the instructions, when executed, further cause the processor to display the visual indicia intraoperatively; (w) wherein the instructions, when executed, further cause the processor to store the visual indicia for use during a future medical procedure; (x) wherein the interior region of the body comprises a kidney, and wherein the instructions, when executed, further cause the processor to: move the instrument into a calyx of the kidney; and tag at least one of: an entrance to the calyx of the kidney, a pole of the kidney, a stone within the kidney, and an area of transitional cell cancer; and/or (y) wherein the instructions, when executed, further cause the processor to: adjust a position determined by the position sensor to account for a physiological movement; and superimposing the adjusted position on the reference image.

In another aspect, a robotic surgical system is disclosed. The robotic surgical system can include: an instrument having an elongate body and at least one position sensor disposed on the elongate body; at least one computer-readable memory having stored thereon executable instructions; and one or more processors in communication with the at least one computer-readable memory and configured to execute the instructions to cause the system to at least: move the instrument within an interior region of a body; receive positional information from the at least one position sensor during the movement of the instrument; display an image of the interior region of the body; and superimpose visual indicia derived from at least a subset of the positional data sets on the image to characterize historical positions of the instrument during the movement of the instrument within the interior region of the body.

In some embodiments, the system can include one or more of the following features in any combination: (a) wherein the position sensor comprises an electromagnetic sensor; (b) wherein the position sensor comprises a shape sensing fiber; (c) wherein the position sensor is positioned on a distal end of the elongate body; (d) wherein the instrument comprises an endoscope; (e) wherein the instrument comprises a ureteroscope; (f) wherein the elongate body is articulable to control a pose of the instrument; (g) an instrument positioning device connected to the instrument, the instrument positioning device configured to manipulate the instrument; (h) wherein the instrument positioning device comprises a robotic arm; (i) wherein the reference image comprises an image captured during a retrograde pyelogram procedure; (j) wherein the reference image comprises a fluoroscopic or an ultrasonic image; (k) wherein the reference image is captured during a computed tomography (CT) or magnetic resonance imaging (MRI) procedure; (l) wherein the instructions, when executed, further cause the one or more processors to capture the reference image intraoperatively; (m) wherein the reference image is captured preoperatively; (n) wherein the instructions, when executed, further cause the one or more processors to: receive instrument image data from an imaging device positioned on the instrument, the instrument image data comprising a plurality of images captured by the imaging device during the movement of the instrument; and for at least a subset of the plurality of images, link each image of the subset with the positional data set indicative of a position at which the image was captured; (o) wherein the instructions, when executed, further cause the one or more processors to store the linked images for use during a future procedure; (p) wherein the instructions, when executed, further cause the processor to: receive a user input of a position selection; and display a linked image corresponding to the user input; (q) wherein the instructions, when executed, further cause the one or more processors to: determine, with the position sensor, a current position of the instrument; and display a linked image corresponding to the determined current position; (r) wherein the instrument image data is captured automatically; (s) wherein the instrument image data is captured upon receipt of a user command; (t) wherein the instructions, when executed, further cause the one or more processors to tag a location or feature of interest within the interior region; (u) wherein tagging comprises receiving a user input; (v) wherein tagging comprises automatically detecting the location or feature of interest; (w) wherein the instructions, when executed, further cause the one or more processors to superimpose the tagged location or feature of interest on the reference image; (x) wherein the instructions, when executed, further cause the one or more processors to connect the visual indicia to characterize the historical path of the movement of the instrument; (y) wherein the visual indicia comprise a mesh; (z) wherein the mesh is indicative of anatomy of the interior region; (aa) wherein the mesh is derived from the subset of the positional data sets and image data received from an imaging device on the instrument; (bb) wherein the subset of the positional data sets is superimposed on the reference image at a durational frequency; (cc) wherein the subset of the positional data sets is superimposed on the reference image at a positional frequency; (dd) wherein the instructions, when executed, further cause the one or more processors to display the visual indicia intraoperatively; (ee) wherein the instructions, when executed, further cause the one or more processors to store the visual indicia for use during a future medical procedure; (ff) wherein the interior region of the body comprises a kidney, and wherein the instructions, when executed, further cause the one or more processors to: move the instrument into a calyx of the kidney; and tag at least one of: an entrance to the calyx of the kidney, a pole of the kidney, a stone within the kidney, and an area of transitional cell cancer; and/or (gg) wherein the instructions, when executed, further cause the one or more processors to: adjust a position determined by the position sensor to account for a physiological movement; and superimpose the adjusted position on the reference image.

In another aspect, a non-transitory computer readable storage medium having stored thereon instructions is described. The instructions, when executed, can cause a processor of a device to at least: move an instrument within an interior region of a body; receive positional information from at least one position sensor of the instrument during movement the instrument, the positional information comprising a plurality of positional data sets, each positional data set indicative of a position of the instrument during the movement of the instrument; receive image data from an imaging device of the instrument within the interior region during the movement of the instrument, the image data comprising one or more images captured by the imaging device at one or more locations within the interior region; link at least a subset of the one or more images with at least a subset of the positional data sets based on the position, as determined by the position sensor, at which each image was captured; determine a user input comprising a position selection; and display a linked image of the linked images corresponding to the position selection.

In some embodiments, the non-transitory computer readable storage medium can include one or more of the following features in any combination: (a) wherein determining the user command comprises receiving the user command; (b) wherein the image data comprises a still image; (c) wherein the image data comprises a video; (d) wherein the subset of the positional data sets is selected at a durational frequency; (e) wherein the subset of the positional data sets is selected at a positional frequency; (f) wherein the positional information comprises information indicative of an orientation of the instrument; (g) wherein the image data is captured automatically; (h) wherein the image data is captured upon receipt of a user command; (i) wherein the instructions, when executed, further cause the processor to: receive a user input associated with a current position; and link the user input with the linked image corresponding to the current position; and/or (j) wherein the instructions, when executed, further cause the processor to detect, within an image of the image data, a feature of interest.

In another aspect a robotic surgical system for navigating an interior region of a body is described. The system can include: an instrument comprising an elongate body, at least one position sensor disposed on the elongate body, and an imaging device disposed on the elongate body; at least one computer-readable memory having stored thereon executable instructions; and one or more processors in communication with the at least one computer-readable memory and configured to execute the instructions to cause the system to at least: move the instrument within an interior region of a body; receive positional information from the at least one position sensor during movement the instrument, the positional information comprising a plurality of positional data sets, each positional data set indicative of a position of the instrument during the movement of the instrument; receive image data from the imaging device, the image data comprising one or more images captured during the movement of the instrument by the imaging device at one or more locations within the interior region; and link at least a subset of the one or more images with at least a subset of the positional data sets based on the position, as determined by the position sensor, at which each image was captured.

In some embodiments, the system can include one or more of the following features in any combination: (a) wherein the position sensor comprises an EM sensor; (b) wherein the position sensor comprises a shape sensing fiber; (c) wherein the position sensor is positioned on a distal end of the elongate body; (d) wherein the instrument comprises an endoscope; (e) wherein the instrument comprises a ureteroscope; (f) wherein the elongate body is articulable to control a pose of the instrument; (g) an instrument positioning device connected to the instrument, the instrument positioning device configured to manipulate the instrument; and/or (h) wherein the instrument positioning device comprises a robotic arm.

In another aspect, a method for navigating an interior region of a body is disclosed. The method can include: moving an instrument within the interior region of the body, the instrument comprising at least one position sensor and at least one imaging device; receiving positional information from the at least one position sensor of the instrument, the positional information comprising a plurality of positional data sets, each positional data set indicative of a position of the instrument during the movement of the instrument; receiving image data from the imaging device of the instrument, the image data comprising one or more images captured by the imaging device at one or more locations within the interior region; linking at least a subset of the one or more images with at least a subset of the positional data sets based on the position, as determined by the position sensor, at which each image was captured; determining, with the at least one position sensor, a current position of the instrument, the current position corresponding a current positional data set of the plurality of positional data sets; and displaying, on a user display, an image linked with the current positional data set.

### BRIEF DESCRIPTION OF THE DRAWINGS

The disclosed aspects will hereinafter be described in conjunction with the appended drawings, provided to illustrate and not to limit the disclosed aspects, wherein like designations denote like elements.
FIG. 1 illustrates an embodiment of a cart-based robotic system arranged for diagnostic and/or therapeutic bronchoscopy procedure(s).
FIG. 2 depicts further aspects of the robotic system of FIG. 1.
FIG. 3 illustrates an embodiment of the robotic system of FIG. 1 arranged for ureteroscopy.
FIG. 4 illustrates an embodiment of the robotic system of FIG. 1 arranged for a vascular procedure.
FIG. 5 illustrates an embodiment of a table-based robotic system arranged for a bronchoscopy procedure.
FIG. 6 provides an alternative view of the robotic system of FIG. 5.
FIG. 7 illustrates an example system configured to stow robotic arm(s).
FIG. 8 illustrates an embodiment of a table-based robotic system configured for a ureteroscopy procedure.
FIG. 9 illustrates an embodiment of a table-based robotic system configured for a laparoscopic procedure.
FIG. 10 illustrates an embodiment of the table-based robotic system of FIGs. 5-9 with pitch or tilt adjustment.
FIG. 11 provides a detailed illustration of the interface between the table and the column of the table-based robotic system of FIGs. 5-10.
FIG. 12 illustrates an exemplary instrument driver.
FIG. 13 illustrates an exemplary medical instrument with a paired instrument driver.
FIG. 14 illustrates an alternative design for an instrument driver and instrument where the axes of the drive units are parallel to the axis of the elongated shaft of the instrument.
FIG. 15 depicts a block diagram illustrating a localization system that estimates a location of one or more elements of the robotic systems of FIGs. 1-10, such as the location of the instrument of FIGs. 13 and 14, in accordance to an example embodiment.
FIG. 16A is a first example fluoroscopic image illustrating an instrument navigating within a kidney.
FIG. 16B is a second example fluoroscopic image illustrating the instrument of FIG. 16A navigating within the kidney.
FIG. 16C is a third example fluoroscopic image illustrating the instrument of FIG. 16A navigating within the kidney.
FIG. 17 illustrates an example representation of visual indicia indicative of positional data received from a position sensor of an instrument navigating within a kidney.
FIG. 18 illustrates the representation of the visual indicia of FIG. 17 superimposed on a reference image of the kidney.
FIG. 19 illustrates that an image captured with an imaging device of a medical instrument navigating within an interior region of a patient may be linked with the position of the medical instrument at which the image was captured.
FIG. 20A is a flowchart illustrating an example method for mapping an interior region of a body.
FIG. 20B is a flowchart illustrating another example method for mapping an interior region of a body.
FIG. 21A is a flowchart illustrating an example method for navigation of an interior region of a body.
FIG. 21B is a flowchart illustrating another example method for navigation of an interior region of a body.
FIG. 22 is a block diagram illustrating certain components of an embodiment of a system for mapping and/or navigation of an interior region of a body.

### DETAILED DESCRIPTION

### 1. Overview.

Aspects of the present disclosure may be integrated into a robotically-enabled medical system capable of performing a variety of medical procedures, including both minimally invasive, such as laparoscopy, and non-invasive, such as endoscopy, procedures. Among endoscopy procedures, the system may be capable of performing bronchoscopy, ureteroscopy, gastroscopy, etc.

In addition to performing the breadth of procedures, the system may provide additional benefits, such as enhanced imaging and guidance to assist the physician. Additionally, the system may provide the physician with the ability to perform the procedure from an ergonomic position without the need for awkward arm motions and positions. Still further, the system may provide the physician with the ability to perform the procedure with improved ease of use such that one or more of the instruments of the system can be controlled by a single user.

Various embodiments will be described below in conjunction with the drawings for purposes of illustration. It should be appreciated that many other implementations of the disclosed concepts are possible, and various advantages can be achieved with the disclosed implementations. Headings are included herein for reference and to aid in locating various sections. These headings are not intended to limit the scope of the concepts described with respect thereto. Such concepts may have applicability throughout the entire specification.

### A. Robotic System - Cart.

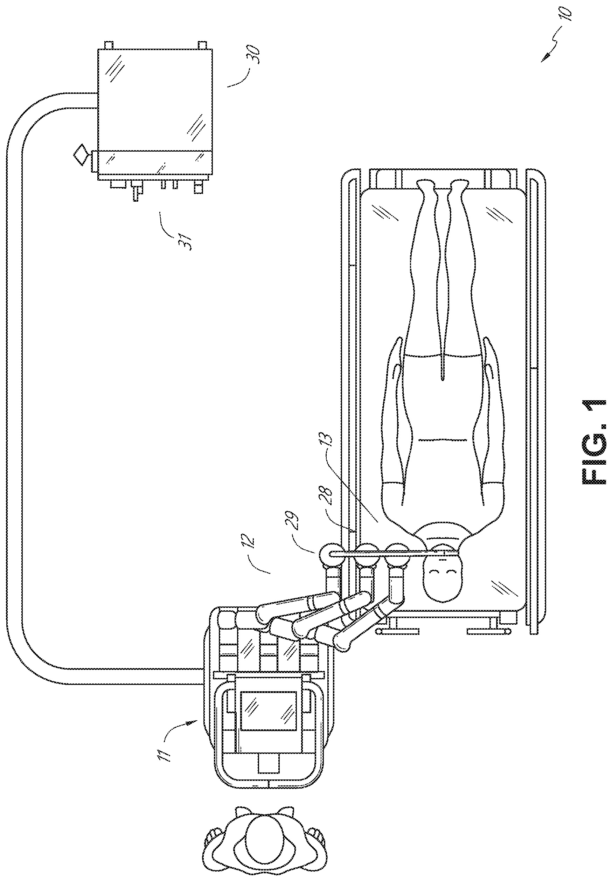

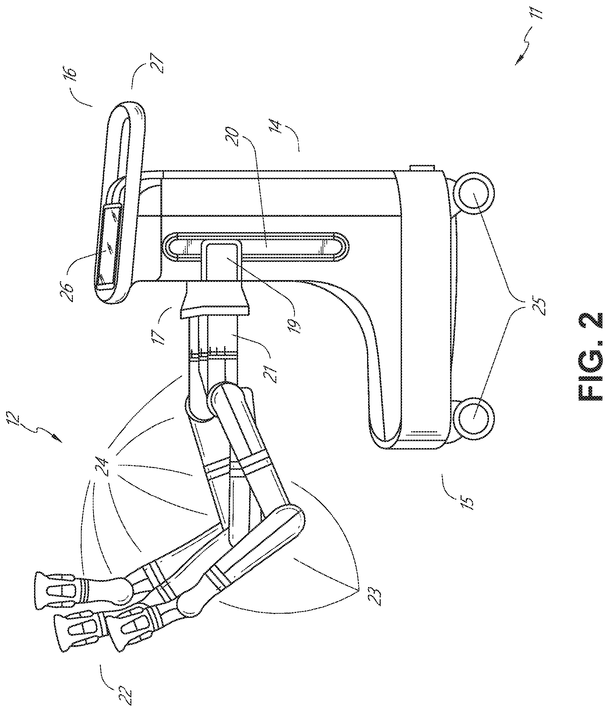

The robotically-enabled medical system may be configured in a variety of ways depending on the particular procedure. FIG. 1 illustrates an embodiment of a cart-based robotically-enabled system 10 arranged for a diagnostic and/or therapeutic bronchoscopy procedure. During a bronchoscopy, the system 10 may comprise a cart 11 having one or more robotic arms 12 to deliver a medical instrument, such as a steerable endoscope 13, which may be a procedure-specific bronchoscope for bronchoscopy, to a natural orifice access point (i.e., the mouth of the patient positioned on a table in the present example) to deliver diagnostic and/or therapeutic tools. As shown, the cart 11 may be positioned proximate to the patient's upper torso in order to provide access to the access point. Similarly, the robotic arms 12 may be actuated to position the bronchoscope relative to the access point. The arrangement in FIG. 1 may also be utilized when performing a gastro-intestinal (GI) procedure with a gastroscope, a specialized endoscope for GI procedures. FIG. 2 depicts an example embodiment of the cart in greater detail.

With continued reference to FIG. 1, once the cart 11 is properly positioned, the robotic arms 12 may insert the steerable endoscope 13 into the patient robotically, manually, or a combination thereof. As shown, the steerable endoscope 13 may comprise at least two telescoping parts, such as an inner leader portion and an outer sheath portion, each portion coupled to a separate instrument driver from the set of instrument drivers 28, each instrument driver coupled to the distal end of an individual robotic arm. This linear arrangement of the instrument drivers 28, which facilitates coaxially aligning the leader portion with the sheath portion, creates a "virtual rail" 29 that may be repositioned in space by manipulating the one or more robotic arms 12 into different angles and/or positions. The virtual rails described herein are depicted in the Figures using dashed lines, and accordingly the dashed lines do not depict any physical structure of the system. Translation of the instrument drivers 28 along the virtual rail 29 telescopes the inner leader portion relative to the outer sheath portion or advances or retracts the endoscope 13 from the patient. The angle of the virtual rail 29 may be adjusted, translated, and pivoted based on clinical application or physician preference. For example, in bronchoscopy, the angle and position of the virtual rail 29 as shown represents a compromise between providing physician access to the endoscope 13 while minimizing friction that results from bending the endoscope 13 into the patient's mouth.

The endoscope 13 may be directed down the patient's trachea and lungs after insertion using precise commands from the robotic system until reaching the target destination or operative site. In order to enhance navigation through the patient's lung network and/or reach the desired target, the endoscope 13 may be manipulated to telescopically extend the inner leader portion from the outer sheath portion to obtain enhanced articulation and greater bend radius. The use of separate instrument drivers 28 also allows the leader portion and sheath portion to be driven independent of each other.

For example, the endoscope 13 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. After identifying a nodule to be malignant, the endoscope 13 may endoscopically deliver tools to resect the potentially cancerous tissue. In some instances, diagnostic and therapeutic treatments may need to be delivered in separate procedures. In those circumstances, the endoscope 13 may also be used to deliver a fiducial to "mark" the location of the target nodule as well. In other instances, diagnostic and therapeutic treatments may be delivered during the same procedure.

The system 10 may also include a movable tower 30, which may be connected via support cables to the cart 11 to provide support for controls, electronics, fluidics, optics, sensors, and/or power to the cart 11. Placing such functionality in the tower 30 allows for a smaller form factor cart 11 that may be more easily adjusted and/or re-positioned by an operating physician and his/her staff. Additionally, the division of functionality between the cart / table and the support tower 30 reduces operating room clutter and facilitates improving clinical workflow. While the cart 11 may be positioned close to the patient, the tower 30 may be stowed in a remote location to stay out of the way during a procedure.

In support of the robotic systems described above, the tower 30 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 30 or the cart 11, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 30 may also include a pump, flow meter, valve control, and/or fluid access in order to provide controlled irrigation and aspiration capabilities to system that may be deployed through the endoscope 13. These components may also be controlled using the computer system of tower 30. In some embodiments, irrigation and aspiration capabilities may be delivered directly to the endoscope 13 through separate cable(s).

The tower 30 may include a voltage and surge protector designed to provide filtered and protected electrical power to the cart 11, thereby avoiding placement of a power transformer and other auxiliary power components in the cart 11, resulting in a smaller, more moveable cart 11.

The tower 30 may also include support equipment for the sensors deployed throughout the robotic system 10. For example, the tower 30 may include opto-electronics equipment for detecting, receiving, and processing data received from the optical sensors or cameras throughout the robotic system 10. In combination with the control system, such opto-electronics equipment may be used to generate real-time images for display in any number of consoles deployed throughout the system, including in the tower 30. Similarly, the tower 30 may also include an electronic subsystem for receiving and processing signals received from deployed electromagnetic (EM) sensors. The tower 30 may also be used to house and position an EM field generator for detection by EM sensors in or on the medical instrument.

The tower 30 may also include a console 31 in addition to other consoles available in the rest of the system, e.g., console mounted on top of the cart. The console 31 may include a user interface and a display screen, such as a touchscreen, for the physician operator. Consoles in system 10 are generally designed to provide both robotic controls as well as pre-operative and real-time information of the procedure, such as navigational and localization information of the endoscope 13. When the console 31 is not the only console available to the physician, it may be used by a second operator, such as a nurse, to monitor the health or vitals of the patient and the operation of system, as well as provide procedure-specific data, such as navigational and localization information.

The tower 30 may be coupled to the cart 11 and endoscope 13 through one or more cables or connections (not shown). In some embodiments, the support functionality from the tower 30 may be provided through a single cable to the cart 11, simplifying and de-cluttering the operating room. In other embodiments, specific functionality may be coupled in separate cabling and connections. For example, while power may be provided through a single power cable to the cart, the support for controls, optics, fluidics, and/or navigation may be provided through a separate cable.

FIG. 2 provides a detailed illustration of an embodiment of the cart from the cart-based robotically-enabled system shown in FIG. 1. The cart 11 generally includes an elongated support structure 14 (often referred to as a "column"), a cart base 15, and a console 16 at the top of the column 14. The column 14 may include one or more carriages, such as a carriage 17 (alternatively "arm support") for supporting the deployment of one or more robotic arms 12 (three shown in FIG. 2). The carriage 17 may include individually configurable arm mounts that rotate along a perpendicular axis to adjust the base of the robotic arms 12 for better positioning relative to the patient. The carriage 17 also includes a carriage interface 19 that allows the carriage 17 to vertically translate along the column 14.

The carriage interface 19 is connected to the column 14 through slots, such as slot 20, that are positioned on opposite sides of the column 14 to guide the vertical translation of the carriage 17. The slot 20 contains a vertical translation interface to position and hold the carriage at various vertical heights relative to the cart base 15. Vertical translation of the carriage 17 allows the cart 11 to adjust the reach of the robotic arms 12 to meet a variety of table heights, patient sizes, and physician preferences. Similarly, the individually configurable arm mounts on the carriage 17 allow the robotic arm base 21 of robotic arms 12 to be angled in a variety of configurations.

In some embodiments, the slot 20 may be supplemented with slot covers that are flush and parallel to the slot surface to prevent dirt and fluid ingress into the internal chambers of the column 14. and the vertical translation interface as the carriage 17 vertically translates. The slot covers may be deployed through pairs of spring spools positioned near the vertical top and bottom of the slot 20. The covers are coiled within the spools until deployed to extend and retract from their coiled state as the carriage 17 vertically translates up and down. The spring-loading of the spools provides force to retract the cover into a spool when carriage 17 translates towards the spool, while also maintaining a tight seal when the carriage 17 translates away from the spool. The covers may be connected to the carriage 17 using, for example, brackets in the carriage interface 19 to ensure proper extension and retraction of the cover as the carriage 17 translates.

The column 14 may internally comprise mechanisms, such as gears and motors, that are designed to use a vertically aligned lead screw to translate the carriage 17 in a mechanized fashion in response to control signals generated in response to user inputs, e.g., inputs from the console 16.

The robotic arms 12 may generally comprise robotic arm bases 21 and end effectors 22, separated by a series of linkages 23 that are connected by a series of joints 24, each joint comprising an independent actuator, each actuator comprising an independently controllable motor. Each independently controllable joint represents an independent degree of freedom available to the robotic arm. Each of the arms 12 have seven joints, and thus provide seven degrees of freedom. A multitude of joints result in a multitude of degrees of freedom, allowing for "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The cart base 15 balances the weight of the column 14, carriage 17, and arms 12 over the floor. Accordingly, the cart base 15 houses heavier components, such as electronics, motors, power supply, as well as components that either enable movement and/or immobilize the cart. For example, the cart base 15 includes rollable wheel-shaped casters 25 that allow for the cart to easily move around the room prior to a procedure. After reaching the appropriate position, the casters 25 may be immobilized using wheel locks to hold the cart 11 in place during the procedure.

Positioned at the vertical end of column 14, the console 16 allows for both a user interface for receiving user input and a display screen (or a dual-purpose device such as, for example, a touchscreen 26) to provide the physician user with both pre-operative and intra-operative data. Potential pre-operative data on the touchscreen 26 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 16 may be positioned and tilted to allow a physician to access the console from the side of the column 14 opposite carriage 17. From this position, the physician may view the console 16, robotic arms 12, and patient while operating the console 16 from behind the cart 11. As shown, the console 16 also includes a handle 27 to assist with maneuvering and stabilizing cart 11.

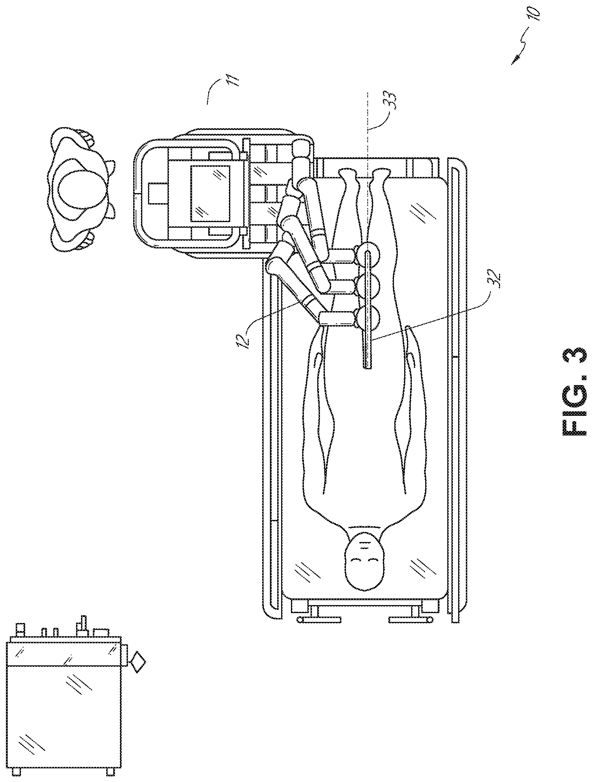

FIG. 3 illustrates an embodiment of a robotically-enabled system 10 arranged for ureteroscopy. In a ureteroscopic procedure, the cart 11 may be positioned to deliver a ureteroscope 32, a procedure-specific endoscope designed to traverse a patient's urethra and ureter, to the lower abdominal area of the patient. In a ureteroscopy, it may be desirable for the ureteroscope 32 to be directly aligned with the patient's urethra to reduce friction and forces on the sensitive anatomy in the area. As shown, the cart 11 may be aligned at the foot of the table to allow the robotic arms 12 to position the ureteroscope 32 for direct linear access to the patient's urethra. From the foot of the table, the robotic arms 12 may insert ureteroscope 32 along the virtual rail 33 directly into the patient's lower abdomen through the urethra.

After insertion into the urethra, using similar control techniques as in bronchoscopy, the ureteroscope 32 may be navigated into the bladder, ureters, and/or kidneys for diagnostic and/or therapeutic applications. For example, the ureteroscope 32 may be directed into the ureter and kidneys to break up kidney stone build up using laser or ultrasonic lithotripsy device deployed down the working channel of the ureteroscope 32. After lithotripsy is complete, the resulting stone fragments may be removed using baskets deployed down the ureteroscope 32.

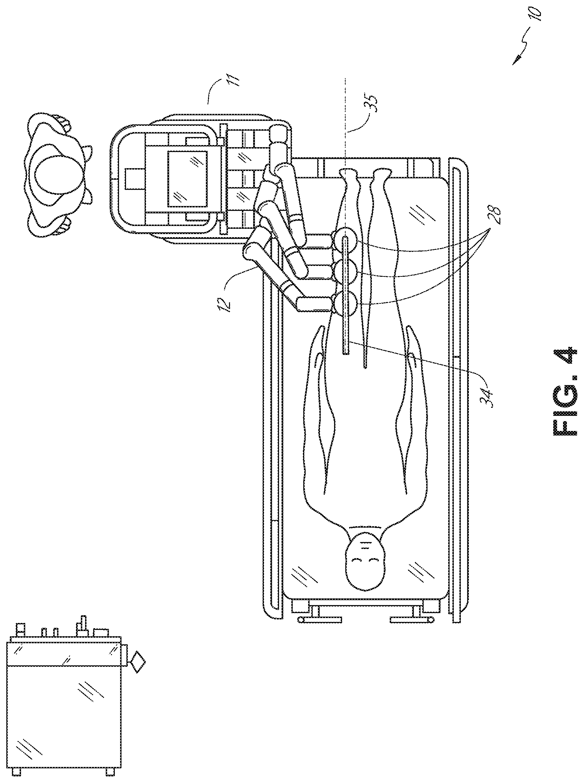

FIG. 4 illustrates an embodiment of a robotically-enabled system similarly arranged for a vascular procedure. In a vascular procedure, the system 10 may be configured such the cart 11 may deliver a medical instrument 34, such as a steerable catheter, to an access point in the femoral artery in the patient's leg. The femoral artery presents both a larger diameter for navigation as well as relatively less circuitous and tortuous path to the patient's heart, which simplifies navigation. As in a ureteroscopic procedure, the cart 11 may be positioned towards the patient's legs and lower abdomen to allow the robotic arms 12 to provide a virtual rail 35 with direct linear access to the femoral artery access point in the patient's thigh / hip region. After insertion into the artery, the medical instrument 34 may be directed and inserted by translating the instrument drivers 28. Alternatively, the cart may be positioned around the patient's upper abdomen in order to reach alternative vascular access points, such as, for example, the carotid and brachial arteries near the shoulder and wrist.

### B. Robotic System - Table.

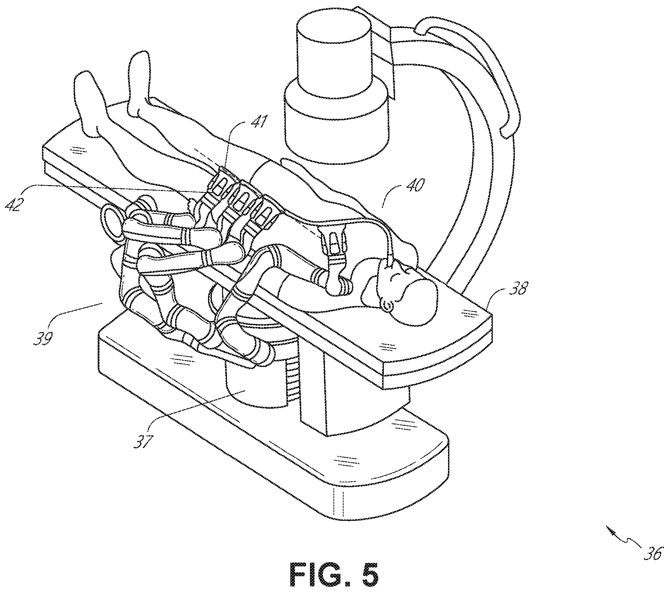

Embodiments of the robotically-enabled medical system may also incorporate the patient's table. Incorporation of the table reduces the amount of capital equipment within the operating room by removing the cart, which allows greater access to the patient. FIG. 5 illustrates an embodiment of such a robotically-enabled system arranged for a bronchoscopy procedure. System 36 includes a support structure or column 37 for supporting platform 38 (shown as a "table" or "bed") over the floor. Much like in the cart-based systems, the end effectors of the robotic arms 39 of the system 36 comprise instrument drivers 42 that are designed to manipulate an elongated medical instrument, such as a bronchoscope 40 in FIG. 5, through or along a virtual rail 41 formed from the linear alignment of the instrument drivers 42. In practice, a C-arm for providing fluoroscopic imaging may be positioned over the patient's upper abdominal area by placing the emitter and detector around table 38.

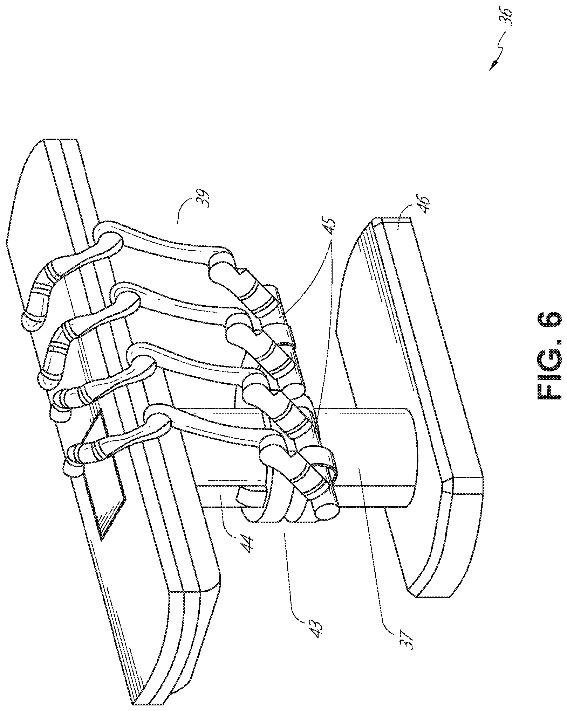

FIG. 6 provides an alternative view of the system 36 without the patient and medical instrument for discussion purposes. As shown, the column 37 may include one or more carriages 43 shown as ring-shaped in the system 36, from which the one or more robotic arms 39 may be based. The carriages 43 may translate along a vertical column interface 44 that runs the length of the column 37 to provide different vantage points from which the robotic arms 39 may be positioned to reach the patient. The carriage(s) 43 may rotate around the column 37 using a mechanical motor positioned within the column 37 to allow the robotic arms 39 to have access to multiples sides of the table 38, such as, for example, both sides of the patient. In embodiments with multiple carriages, the carriages may be individually positioned on the column and may translate and/or rotate independent of the other carriages. While carriages 43 need not surround the column 37 or even be circular, the ring-shape as shown facilitates rotation of the carriages 43 around the column 37 while maintaining structural balance. Rotation and translation of the carriages 43 allows the system to align the medical instruments, such as endoscopes and laparoscopes, into different access points on the patient.

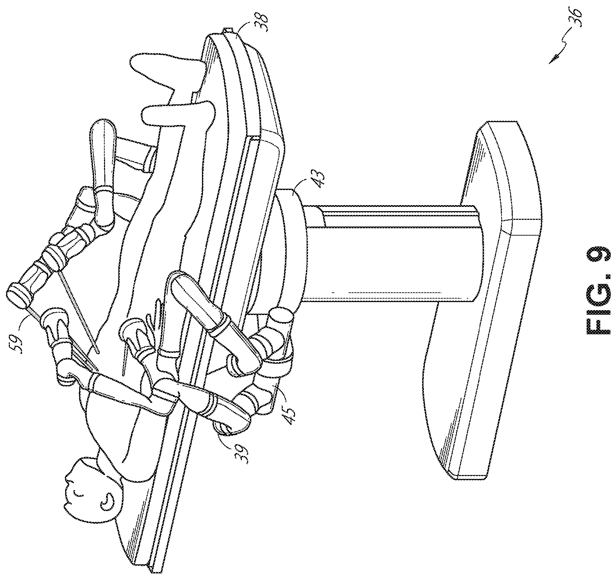

The arms 39 may be mounted on the carriages through a set of arm mounts 45 comprising a series of joints that may individually rotate and/or telescopically extend to provide additional configurability to the robotic arms 39. Additionally, the arm mounts 45 may be positioned on the carriages 43 such that, when the carriages 43 are appropriately rotated, the arm mounts 45 may be positioned on either the same side of table 38 (as shown in FIG. 6), on opposite sides of table 38 (as shown in FIG. 9), or on adjacent sides of the table 38 (not shown).

The column 37 structurally provides support for the table 38, and a path for vertical translation of the carriages. Internally, the column 37 may be equipped with lead screws for guiding vertical translation of the carriages, and motors to mechanize the translation of said carriages based the lead screws. The column 37 may also convey power and control signals to the carriage 43 and robotic arms 39 mounted thereon.

The table base 46 serves a similar function as the cart base 15 in cart 11 shown in FIG. 2, housing heavier components to balance the table/bed 38, the column 37, the carriages 43, and the robotic arms 39. The table base 46 may also incorporate rigid casters to provide stability during procedures. Deployed from the bottom of the table base 46, the casters may extend in opposite directions on both sides of the base 46 and retract when the system 36 needs to be moved.

Continuing with FIG. 6, the system 36 may also include a tower (not shown) that divides the functionality of system 36 between table and tower to reduce the form factor and bulk of the table. As in earlier disclosed embodiments, the tower may provide a variety of support functionalities to table, such as processing, computing, and control capabilities, power, fluidics, and/or optical and sensor processing. The tower may also be movable to be positioned away from the patient to improve physician access and de-clutter the operating room. Additionally, placing components in the tower allows for more storage space in the table base for potential stowage of the robotic arms. The tower may also include a console that provides both a user interface for user input, such as keyboard and/or pendant, as well as a display screen (or touchscreen) for pre-operative and intra-operative information, such as real-time imaging, navigation, and tracking information.

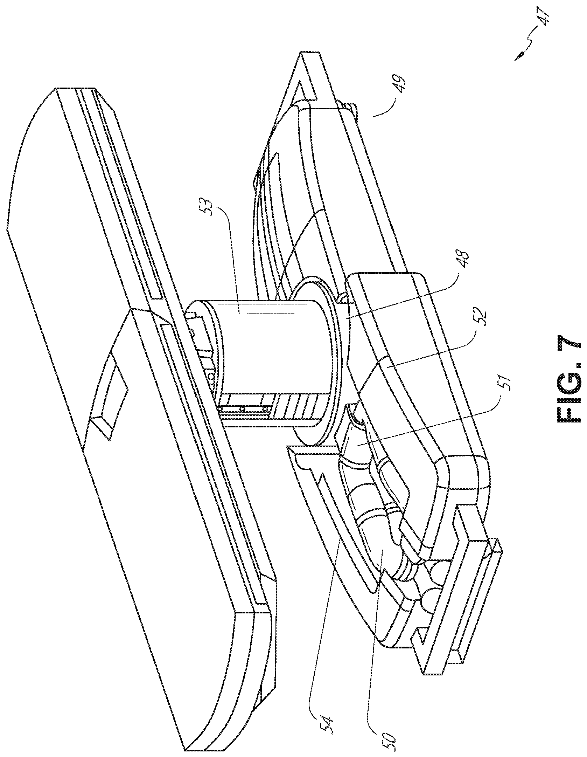

In some embodiments, a table base may stow and store the robotic arms when not in use. FIG. 7 illustrates a system 47 that stows robotic arms in an embodiment of the table-based system. In system 47, carriages 48 may be vertically translated into base 49 to stow robotic arms 50, arm mounts 51, and the carriages 48 within the base 49. Base covers 52 may be translated and retracted open to deploy the carriages 48, arm mounts 51, and arms 50 around column 53, and closed to stow to protect them when not in use. The base covers 52 may be sealed with a membrane 54 along the edges of its opening to prevent dirt and fluid ingress when closed.

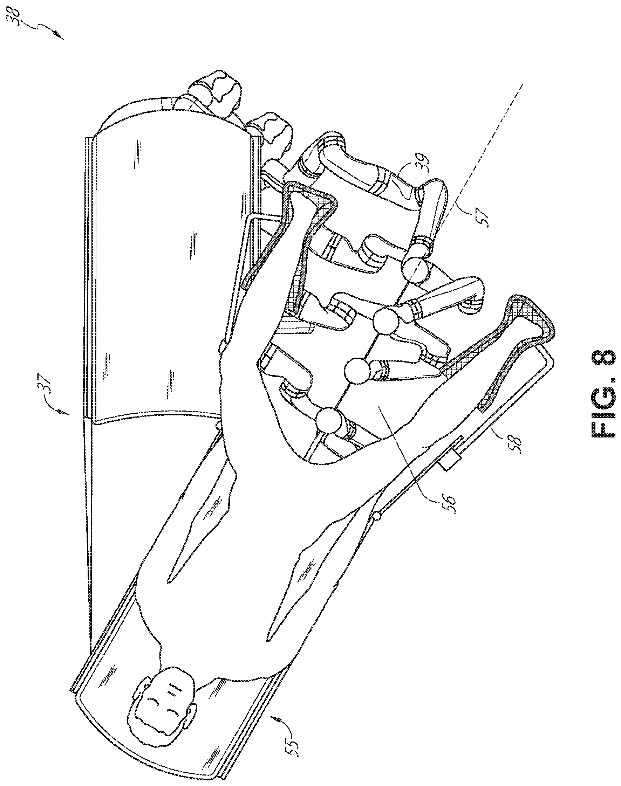

FIG. 8 illustrates an embodiment of a robotically-enabled table-based system configured for a ureteroscopy procedure. In a ureteroscopy, the table 38 may include a swivel portion 55 for positioning a patient off-angle from the column 37 and table base 46. The swivel portion 55 may rotate or pivot around a pivot point (e.g., located below the patient's head) in order to position the bottom portion of the swivel portion 55 away from the column 37. For example, the pivoting of the swivel portion 55 allows a C-arm (not shown) to be positioned over the patient's lower abdomen without competing for space with the column (not shown) below table 38. By rotating the carriage 35 (not shown) around the column 37, the robotic arms 39 may directly insert a ureteroscope 56 along a virtual rail 57 into the patient's groin area to reach the urethra. In a ureteroscopy, stirrups 58 may also be fixed to the swivel portion 55 of the table 38 to support the position of the patient's legs during the procedure and allow clear access to the patient's groin area.

In a laparoscopic procedure, through small incision(s) in the patient's abdominal wall, minimally invasive instruments (elongated in shape to accommodate the size of the one or more incisions) may be inserted into the patient's anatomy. After inflation of the patient's abdominal cavity, the instruments, often referred to as laparoscopes, may be directed to perform surgical tasks, such as grasping, cutting, ablating, suturing, etc. FIG. 9 illustrates an embodiment of a robotically-enabled table-based system configured for a laparoscopic procedure. As shown in FIG. 9, the carriages 43 of the system 36 may be rotated and vertically adjusted to position pairs of the robotic arms 39 on opposite sides of the table 38, such that laparoscopes 59 may be positioned using the arm mounts 45 to be passed through minimal incisions on both sides of the patient to reach his/her abdominal cavity.

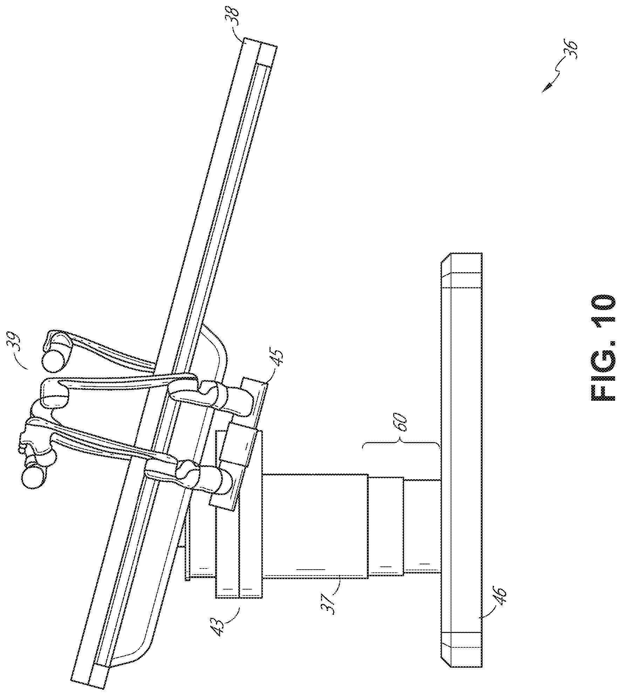

To accommodate laparoscopic procedures, the robotically-enabled table system may also tilt the platform to a desired angle. FIG. 10 illustrates an embodiment of the robotically-enabled medical system with pitch or tilt adjustment. As shown in FIG. 10, the system 36 may accommodate tilt of the table 38 to position one portion of the table at a greater distance from the floor than the other. Additionally, the arm mounts 45 may rotate to match the tilt such that the arms 39 maintain the same planar relationship with table 38. To accommodate steeper angles, the column 37 may also include telescoping portions 60 that allow vertical extension of column 37 to keep the table 38 from touching the floor or colliding with base 46.

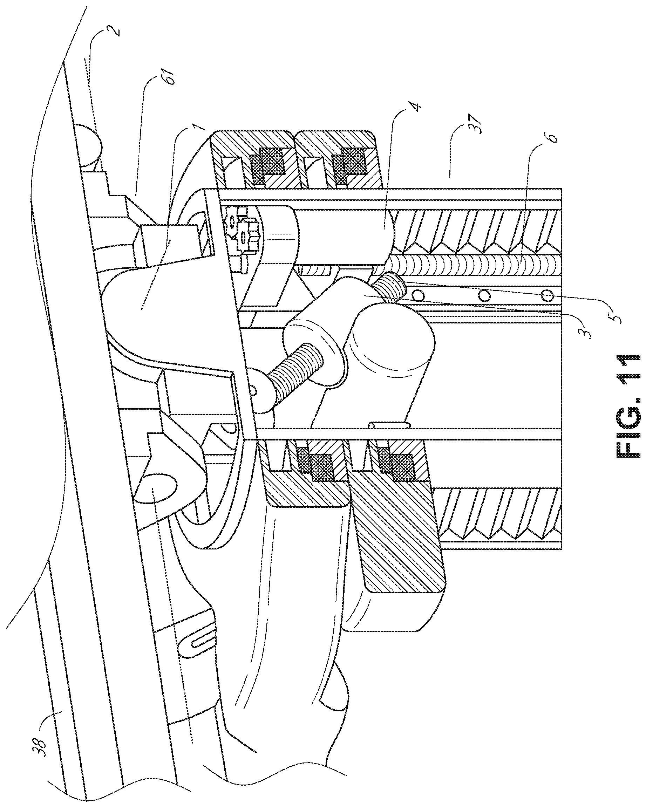

FIG. 11 provides a detailed illustration of the interface between the table 38 and the column 37. Pitch rotation mechanism 61 may be configured to alter the pitch angle of the table 38 relative to the column 37 in multiple degrees of freedom. The pitch rotation mechanism 61 may be enabled by the positioning of orthogonal axes 1, 2 at the column-table interface, each axis actuated by a separate motor 3, 4 responsive to an electrical pitch angle command. Rotation along one screw 5 would enable tilt adjustments in one axis 1, while rotation along the other screw 6 would enable tilt adjustments along the other axis 2.

For example, pitch adjustments are particularly useful when trying to position the table in a Trendelenburg position, i.e., position the patient's lower abdomen at a higher position from the floor than the patient's lower abdomen, for lower abdominal surgery. The Trendelenburg position causes the patient's internal organs to slide towards his/her upper abdomen through the force of gravity, clearing out the abdominal cavity for minimally invasive tools to enter and perform lower abdominal surgical procedures, such as laparoscopic prostatectomy.

### C. Instrument Driver & Interface.

The end effectors of the system's robotic arms comprise (i) an instrument driver (alternatively referred to as "instrument drive mechanism" or "instrument device manipulator") that incorporate electro-mechanical means for actuating the medical instrument and (ii) a removable or detachable medical instrument which may be devoid of any electro-mechanical components, such as motors. This dichotomy may be driven by the need to sterilize medical instruments used in medical procedures, and the inability to adequately sterilize expensive capital equipment due to their intricate mechanical assemblies and sensitive electronics. Accordingly, the medical instruments may be designed to be detached, removed, and interchanged from the instrument driver (and thus the system) for individual sterilization or disposal by the physician or the physician's staff. In contrast, the instrument drivers need not be changed or sterilized, and may be draped for protection.

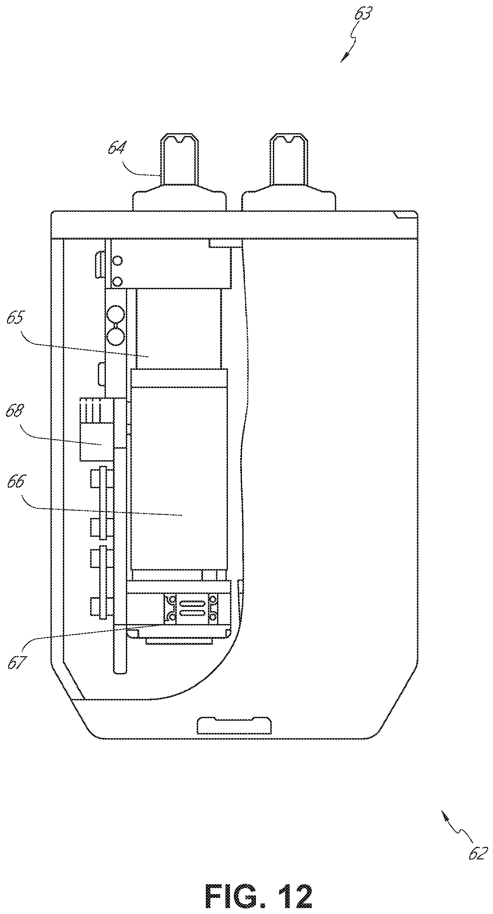

FIG. 12 illustrates an example instrument driver. Positioned at the distal end of a robotic arm, instrument driver 62 comprises of one or more drive units 63 arranged with parallel axes to provide controlled torque to a medical instrument via drive shafts 64. Each drive unit 63 comprises an individual drive shaft 64 for interacting with the instrument, a gear head 65 for converting the motor shaft rotation to a desired torque, a motor 66 for generating the drive torque, an encoder 67 to measure the speed of the motor shaft and provide feedback to the control circuitry, and control circuity 68 for receiving control signals and actuating the drive unit. Each drive unit 63 being independent controlled and motorized, the instrument driver 62 may provide multiple (four as shown in FIG. 12) independent drive outputs to the medical instrument. In operation, the control circuitry 68 would receive a control signal, transmit a motor signal to the motor 66, compare the resulting motor speed as measured by the encoder 67 with the desired speed, and modulate the motor signal to generate the desired torque.

For procedures that require a sterile environment, the robotic system may incorporate a drive interface, such as a sterile adapter connected to a sterile drape, that sits between the instrument driver and the medical instrument. The chief purpose of the sterile adapter is to transfer angular motion from the drive shafts of the instrument driver to the drive inputs of the instrument while maintaining physical separation, and thus sterility, between the drive shafts and drive inputs. Accordingly, an example sterile adapter may comprise of a series of rotational inputs and outputs intended to be mated with the drive shafts of the instrument driver and drive inputs on the instrument. Connected to the sterile adapter, the sterile drape, comprised of a thin, flexible material such as transparent or translucent plastic, is designed to cover the capital equipment, such as the instrument driver, robotic arm, and cart (in a cart-based system) or table (in a table-based system). Use of the drape would allow the capital equipment to be positioned proximate to the patient while still being located in an area not requiring sterilization (i.e., non-sterile field). On the other side of the sterile drape, the medical instrument may interface with the patient in an area requiring sterilization (i.e., sterile field).

### D. Medical Instrument.

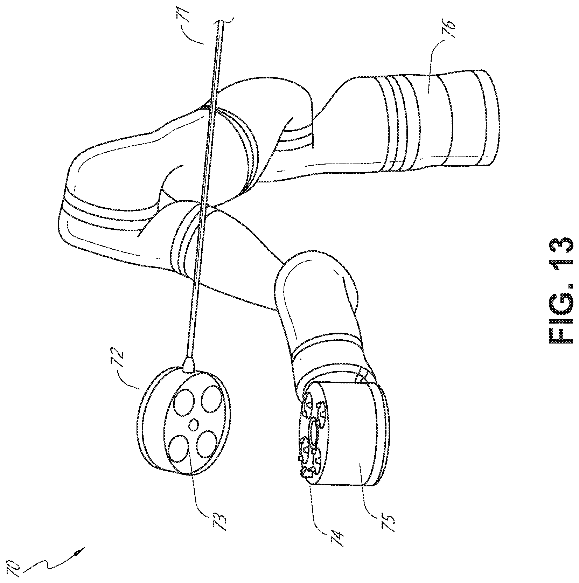

FIG. 13 illustrates an example medical instrument with a paired instrument driver. Like other instruments designed for use with a robotic system, medical instrument 70 comprises an elongated shaft 71 (or elongate body) and an instrument base 72. The instrument base 72, also referred to as an "instrument handle" due to its intended design for manual interaction by the physician, may generally comprise rotatable drive inputs 73, e.g., receptacles, pulleys or spools, that are designed to be mated with drive outputs 74 that extend through a drive interface on instrument driver 75 at the distal end of robotic arm 76. When physically connected, latched, and/or coupled, the mated drive inputs 73 of instrument base 72 may share axes of rotation with the drive outputs 74 in the instrument driver 75 to allow the transfer of torque from drive outputs 74 to drive inputs 73. In some embodiments, the drive outputs 74 may comprise splines that are designed to mate with receptacles on the drive inputs 73.

The elongated shaft 71 is designed to be delivered through either an anatomical opening or lumen, e.g., as in endoscopy, or a minimally invasive incision, e.g., as in laparoscopy. The elongated shaft 66 may be either flexible (e.g., having properties similar to an endoscope) or rigid (e.g., having properties similar to a laparoscope) or contain a customized combination of both flexible and rigid portions. When designed for laparoscopy, the distal end of a rigid elongated shaft may be connected to an end effector comprising a jointed wrist formed from a clevis with an axis of rotation and a surgical tool, such as, for example, a grasper or scissors, that may be actuated based on force from the tendons as the drive inputs rotate in response to torque received from the drive outputs 74 of the instrument driver 75. When designed for endoscopy, the distal end of a flexible elongated shaft may include a steerable or controllable bending section that may be articulated and bent based on torque received from the drive outputs 74 of the instrument driver 75.

Torque from the instrument driver 75 is transmitted down the elongated shaft 71 using tendons within the shaft 71. These individual tendons, such as pull wires, may be individually anchored to individual drive inputs 73 within the instrument handle 72. From the handle 72, the tendons are directed down one or more pull lumens within the elongated shaft 71 and anchored at the distal portion of the elongated shaft 71. In laparoscopy, these tendons may be coupled to a distally mounted end effector, such as a wrist, grasper, or scissor. Under such an arrangement, torque exerted on drive inputs 73 would transfer tension to the tendon, thereby causing the end effector to actuate in some way. In laparoscopy, the tendon may cause a joint to rotate about an axis, thereby causing the end effector to move in one direction or another. Alternatively, the tendon may be connected to one or more jaws of a grasper at distal end of the elongated shaft 71, where tension from the tendon cause the grasper to close.

In endoscopy, the tendons may be coupled to a bending or articulating section positioned along the elongated shaft 71 (e.g., at the distal end) via adhesive, control ring, or other mechanical fixation. When fixedly attached to the distal end of a bending section, torque exerted on drive inputs 73 would be transmitted down the tendons, causing the softer, bending section (sometimes referred to as the articulable section or region) to bend or articulate. Along the non-bending sections, it may be advantageous to spiral or helix the individual pull lumens that direct the individual tendons along (or inside) the walls of the endoscope shaft to balance the radial forces that result from tension in the pull wires. The angle of the spiraling and/or spacing there between may be altered or engineered for specific purposes, wherein tighter spiraling exhibits lesser shaft compression under load forces, while lower amounts of spiraling results in greater shaft compression under load forces, but also exhibits limits bending. On the other end of the spectrum, the pull lumens may be directed parallel to the longitudinal axis of the elongated shaft 71 to allow for controlled articulation in the desired bending or articulable sections.

In endoscopy, the elongated shaft 71 houses a number of components to assist with the robotic procedure. The shaft may comprise of a working channel for deploying surgical tools, irrigation, and/or aspiration to the operative region at the distal end of the shaft 71. The shaft 71 may also accommodate wires and/or optical fibers to transfer signals to/from an optical assembly at the distal tip, which may include of an optical camera. The shaft 71 may also accommodate optical fibers to carry light from proximally-located light sources, such as light emitting diodes, to the distal end of the shaft.

At the distal end of the instrument 70, the distal tip may also comprise the opening of a working channel for delivering tools for diagnostic and/or therapy, irrigation, and aspiration to an operative site. The distal tip may also include a port for a camera, such as a fiberscope or a digital camera, to capture images of an internal anatomical space. Relatedly, the distal tip may also include ports for light sources for illuminating the anatomical space when using the camera.

In the example of FIG. 13, the drive shaft axes, and thus the drive input axes, are orthogonal to the axis of the elongated shaft. This arrangement, however, complicates roll capabilities for the elongated shaft 71. Rolling the elongated shaft 71 along its axis while keeping the drive inputs 73 static results in undesirable tangling of the tendons as they extend off the drive inputs 73 and enter pull lumens within the elongate shaft 71. The resulting entanglement of such tendons may disrupt any control algorithms intended to predict movement of the flexible elongate shaft during an endoscopic procedure.

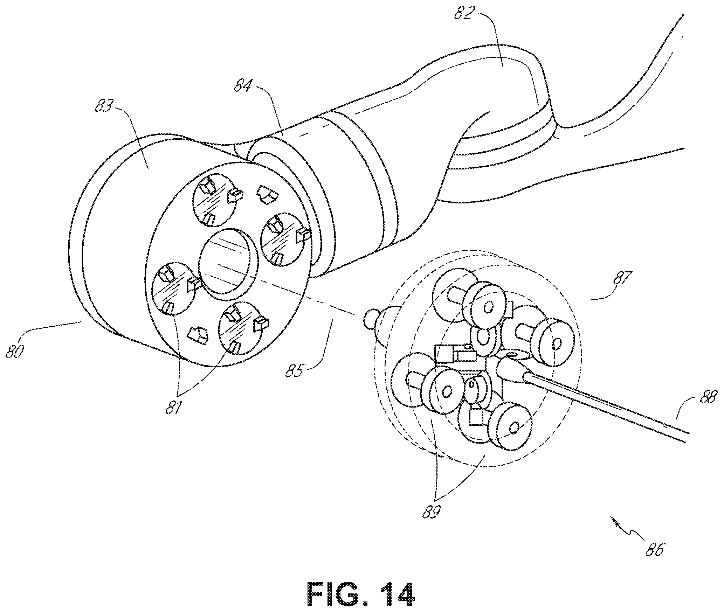

FIG. 14 illustrates an alternative design for an instrument driver and instrument where the axes of the drive units are parallel to the axis of the elongated shaft of the instrument. As shown, a circular instrument driver 80 comprises four drive units with their drive outputs 81 aligned in parallel at the end of a robotic arm 82. The drive units, and their respective drive outputs 81, are housed in a rotational assembly 83 of the instrument driver 80 that is driven by one of the drive units within the assembly 83. In response to torque provided by the rotational drive unit, the rotational assembly 83 rotates along a circular bearing that connects the rotational assembly 83 to the non-rotational portion 84 of the instrument driver. Power and controls signals may be communicated from the non-rotational portion 84 of the instrument driver 80 to the rotational assembly 83 through electrical contacts may be maintained through rotation by a brushed slip ring connection (not shown). In other embodiments, the rotational assembly 83 may be responsive to a separate drive unit that is integrated into the non-rotatable portion 84, and thus not in parallel to the other drive units. The rotational mechanism 83 allows the instrument driver 80 to rotate the drive units, and their respective drive outputs 81, as a single unit around an instrument driver axis 85.

Like earlier disclosed embodiments, an instrument 86 may comprise of an elongated shaft portion 88 and an instrument base 87 (shown with a transparent external skin for discussion purposes) comprising a plurality of drive inputs 89 (such as receptacles, pulleys, and spools) that are configured to receive the drive outputs 81 in the instrument driver 80. Unlike prior disclosed embodiments, instrument shaft 88 extends from the center of instrument base 87 with an axis substantially parallel to the axes of the drive inputs 89, rather than orthogonal as in the design of FIG. 13.

When coupled to the rotational assembly 83 of the instrument driver 80, the medical instrument 86, comprising instrument base 87 and instrument shaft 88, rotates in combination with the rotational assembly 83 about the instrument driver axis 85. Since the instrument shaft 88 is positioned at the center of instrument base 87, the instrument shaft 88 is coaxial with instrument driver axis 85 when attached. Thus, rotation of the rotational assembly 83 causes the instrument shaft 88 to rotate about its own longitudinal axis. Moreover, as the instrument base 87 rotates with the instrument shaft 88, any tendons connected to the drive inputs 89 in the instrument base 87 are not tangled during rotation. Accordingly, the parallelism of the axes of the drive outputs 81, drive inputs 89, and instrument shaft 88 allows for the shaft rotation without tangling any control tendons.

### E. Navigation and Control.

Traditional endoscopy may involve the use of fluoroscopy (e.g., as may be delivered through a C-arm) and other forms of radiation-based imaging modalities to provide endoluminal guidance to an operator physician. In contrast, the robotic systems contemplated by this disclosure can provide for non-radiation-based navigational and localization means to reduce physician exposure to radiation and reduce the amount of equipment within the operating room. As used herein, the term "localization" may refer to determining and/or monitoring the position of objects in a reference coordinate system. Technologies such as pre-operative mapping, computer vision, real-time EM tracking, and robot command data may be used individually or in combination to achieve a radiation-free operating environment. In other cases, where radiation-based imaging modalities are still used, the pre-operative mapping, computer vision, real-time EM tracking, and robot command data may be used individually or in combination to improve upon the information obtained solely through radiation-based imaging modalities.

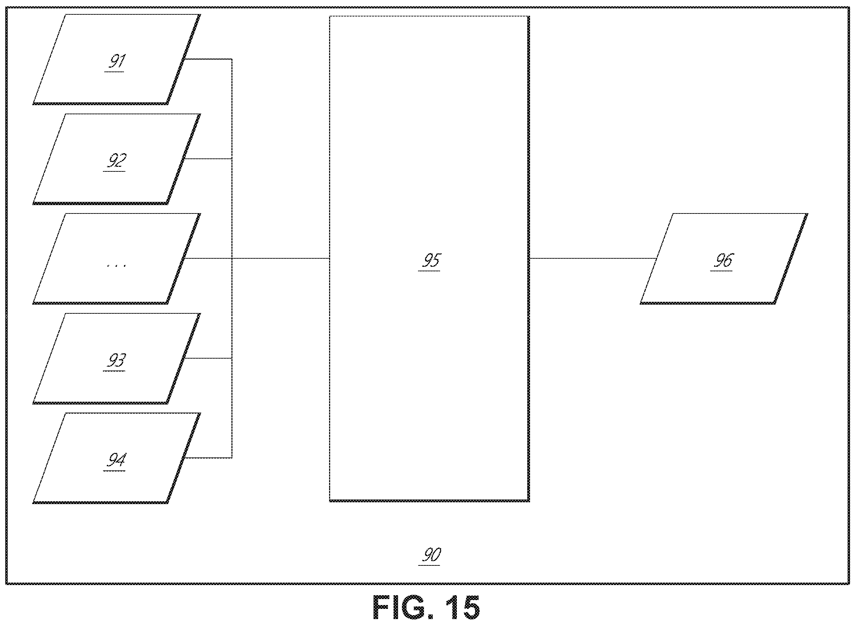

FIG. 15 is a block diagram illustrating a localization system 90 that estimates a location of one or more elements of the robotic system, such as the location of the instrument, in accordance to an example embodiment. The localization system 90 may be a set of one or more computer devices configured to execute one or more instructions. The computer devices may be embodied by a processor (or processors) and computer-readable memory in one or more components discussed above. By way of example and not limitation, the computer devices may be in the tower 30 shown in FIG. 1, the cart shown in FIGs. 1-4, the beds shown in FIGs. 5-10, etc.

As shown in FIG. 15, the localization system 90 may include a localization module 95 that processes input data 91-94 to generate location data 96 for the distal tip of a medical instrument. The location data 96 may be data or logic that represents a location and/or orientation of the distal end of the instrument relative to a frame of reference. The frame of reference can be a frame of reference relative to the anatomy of the patient or to a known object, such as an EM field generator (see discussion below for the EM field generator).

The various input data 91-94 are now described in greater detail. Pre-operative mapping may be accomplished through the use of the collection of low dose CT scans. Pre-operative CT scans are reconstructed into three-dimensional images, which are visualized, e.g., as "slices" of a cutaway view of the patient's internal anatomy. When analyzed in the aggregate, image-based models for anatomical cavities, spaces and structures of the patient's anatomy, such as a patient lung network, may be generated. Techniques such as center-line geometry may be determined and approximated from the CT images to develop a three-dimensional volume of the patient's anatomy, referred to as preoperative model data 91. The use of center-line geometry is discussed in U.S. Pat. App. No. 14/523,760. Network topological models may also be derived from the CT-images, and are particularly appropriate for bronchoscopy.

In some embodiments, the instrument may be equipped with a camera to provide vision data 92. The localization module 95 may process the vision data to enable one or more vision-based location tracking. For example, the preoperative model data may be used in conjunction with the vision data 92 to enable computer vision-based tracking of the medical instrument (e.g., an endoscope or an instrument advance through a working channel of the endoscope). For example, using the preoperative model data 91, the robotic system may generate a library of expected endoscopic images from the model based on the expected path of travel of the endoscope, each image linked to a location within the model. Intra-operatively, this library may be referenced by the robotic system in order to compare real-time images captured at the camera (e.g., a camera at a distal end of the endoscope) to those in the image library to assist localization.

Other computer vision-based tracking techniques use feature tracking to determine motion of the camera, and thus the endoscope. Some feature of the localization module 95 may identify circular geometries in the preoperative model data 91 that correspond to anatomical lumens and track the change of those geometries to determine which anatomical lumen was selected, as well as the relative rotational and/or translational motion of the camera. Use of a topological map may further enhance vision-based algorithms or techniques.

Optical flow, another computer vision-based technique, may analyze the displacement and translation of image pixels in a video sequence in the vision data 92 to infer camera movement. Examples of optical flow techniques may include motion detection, object segmentation calculations, luminance, motion compensated encoding, stereo disparity measurement, etc. Through the comparison of multiple frames over multiple iterations, movement and location of the camera (and thus the endoscope) may be determined.

The localization module 95 may use real-time EM tracking to generate a real-time location of the endoscope in a global coordinate system that may be registered to the patient's anatomy, represented by the preoperative model. In EM tracking, an EM sensor (or tracker) comprising of one or more sensor coils embedded in one or more locations and orientations in a medical instrument (e.g., an endoscopic tool) measures the variation in the EM field created by one or more static EM field generators positioned at a known location. The location information detected by the EM sensors is stored as EM data 93. The EM field generator (or transmitter), may be placed close to the patient to create a low intensity magnetic field that the embedded sensor may detect. The magnetic field induces small currents in the sensor coils of the EM sensor, which may be analyzed to determine the distance and angle between the EM sensor and the EM field generator. These distances and orientations may be intra-operatively "registered" to the patient anatomy (e.g., the preoperative model) in order to determine the geometric transformation that aligns a single location in the coordinate system with a position in the pre-operative model of the patient's anatomy. Once registered, an embedded EM tracker in one or more positions of the medical instrument (e.g., the distal tip of an endoscope) may provide real-time indications of the progression of the medical instrument through the patient's anatomy.

Robotic command and kinematics data 94 may also be used by the localization module 95 to provide localization data 96 for the robotic system. Device pitch and yaw resulting from articulation commands may be determined during pre-operative calibration. Intra-operatively, these calibration measurements may be used in combination with known insertion depth information to estimate the position of the instrument. Alternatively, these calculations may be analyzed in combination with EM, vision, and/or topological modeling to estimate the position of the medical instrument within the network.

As FIG. 15 shows, a number of other input data can be used by the localization module 95. For example, although not shown in FIG. 15, an instrument utilizing shape-sensing fiber can provide shape data that the localization module 95 can use to determine the location and shape of the instrument.

The localization module 95 may use the input data 91-94 in combination(s). In some cases, such a combination may use a probabilistic approach where the localization module 95 assigns a confidence weight to the location determined from each of the input data 91-94. Thus, where the EM data may not be reliable (as may be the case where there is EM interference) the confidence of the location determined by the EM data 93 can be decrease and the localization module 95 may rely more heavily on the vision data 92 and/or the robotic command and kinematics data 94.

As discussed above, the robotic systems discussed herein may be designed to incorporate a combination of one or more of the technologies above. The robotic system's computer-based control system, based in the tower, bed and/or cart, may store computer program instructions, for example, within a non-transitory computer-readable storage medium such as a persistent magnetic storage drive, solid state drive, or the like, that, upon execution, cause the system to receive and analyze sensor data and user commands, generate control signals throughout the system, and display the navigational and localization data, such as the position of the instrument within the global coordinate system, anatomical map, etc.

### 2. Mapping and Navigation of an Interior Region of a Body.

Embodiments of the disclosure relate to systems and techniques for mapping and/or navigation of an interior region of a body with a robotically-enabled medical instrument. As will be described in greater detail below, the systems and techniques may be configured to generate visual indicia indicative of previous positions of the instrument as the instrument is navigated through the interior region. The visual indicia may be superimposed on a reference image of the interior region and displayed to the user. The visual indicia may be derived from positional information received from a position sensor. The position sensor may be positioned on the instrument. The visual indicia may form a map representative of the previous positions of the instrument. The map can be used to visualize the anatomy of the interior region and can be used by a physician to navigate the instrument within the interior region. For example, the physician may use the map created by the visual indicia to navigate to a previously visited portion of the interior region or to determine when the instrument is being navigated into a new (i.e., not previously visited) portion of the interior region.

The methods and techniques may also be configured to receive image data from an image sensor positioned on the instrument. The image data can include still images or videos. The methods and techniques can link the image data with the positional data, such that an image captured at a particular location can be recalled and displayed to the user. In some implementations, images are recalled and displayed when a user selects a position or when the instrument is positioned at a position for which there is a linked image.

Further, the methods and techniques may be configured to permit the physician to tag certain features or locations of interest.

The systems and techniques for mapping and/or navigation of an interior region of a body with the robotically-enabled medical instrument can be employed during many medical procedures, such as endoscopic or laparoscopic procedures. The systems and techniques can improve upon conventional techniques by reducing or eliminating one or more complications or challenges associated with the conventional techniques as described below. In some embodiments, the techniques described herein can be used in manual medical procedures.

In many of the examples described herein, the systems and techniques for mapping and/or navigation of an interior region of a body are described with reference to a ureteroscopic procedure for removing kidney stones from a kidney. It will be appreciated, however, that the systems and techniques can be employed in other medical procedures in which a medical instrument is navigated within an interior region of a patient's body, such as bronchoscopy, gastroscopy, and others.

### A. Introduction.

At the beginning and end of ureteroscopic or percutaneous kidney stone removal procedures, physicians often perform a mapping step. This may involve navigating a flexible endoscope, such as a ureteroscope or cystoscope, to the first (cephalad) calyx, visually observing for stone fragments with an imaging device on the endoscope, and then sequentially moving to the next calyx until the bottom most calyx has been reached. Physicians use this systematic approach in an effort to ensure that all areas of the kidney have been observed despite the wide variation in kidney morphology.

There can be several challenges with this mapping procedure. First, such mapping may be time intensive. For example, whenever a fragment is found and treated, the physician needs to repeat the mapping process, starting again from the first calyx. Second, such mapping may be radiation intensive. For example, it is often unclear where the endoscope is located inside the kidney based on the endoscopic view alone. Thus, physicians often take fluoroscopic images, and sometimes a selective pyelogram, at every calyx in an effort to verify the position of the endoscope within the kidney. Third, such mapping can be error prone. The rate of residual fragments suggest that physicians often miss calyces during this mapping procedure.

Some embodiments discussed herein may relate to systems and techniques for mapping and/or navigation of an interior region of a body (e.g., a kidney or others) with a robotically-enabled medical instrument (e.g., an endoscope, a ureteroscope, a cystoscope, etc.) that can improve upon the mapping technique discussed above and/or reduce or eliminate one or more of the associated challenges. For example, in some implementations, the systems and techniques for mapping and/or navigation discussed herein may increase a physician's confidence that she has navigated the instrument through every calyx of the kidney, while reducing the amount of radiation and the procedure time required for the mapping.

In some implementations, the systems and techniques for mapping and/or navigation may provide the physician with continuous tracking of the instrument's position with a map-like context of the anatomy. Further, in some implementations tagging of images taken with an imaging device on the endoscope and locations of interest further enhance the ability to inform the physician of where the instrument is currently located inside the anatomy.

For example, a flexible ureteroscope or a flexible cystoscope including an EM sensor (or other position sensor) built into its distal tip (or elsewhere on the scope) can be inserted through a working channel of an endoscope that is used to navigate a kidney. The EM sensor can provide positional data indicative of the position (e.g., location and/or orientation) of the instrument. As will be described in greater detail below, other sensors and methods for determining the position of the instrument (e.g., shape-sensing fibers, impedance tracking, or other forms of localization) can be used in addition to or in place of the EM sensor. Visual indicia derived from the positional information can be displayed to the physician. In some implementations, the visual indicia are superimposed on top of a reference image (e.g., a preoperative CT image or an intraoperative or preoperative retrograde pyelogram) of the anatomy. The positional data can be registered, for example with a one, two, or three-point registration, to align a coordinate system of the positional data with a coordinate system of the reference image.

Registration of the coordinate system of the positional data with the coordinate system of the reference image can be accomplished in a variety of ways. As one example, the scope can be navigated to one or more features within the anatomy that can be recognized from the point of view of the scope (e.g., in images captured with an imaging device on the scope) and/or in the reference image, such as, for example, both from the point of view of the scope and in the reference image. Positional data from the scope, when positioned at the recognized anatomical features, can be used to align the coordinate system of the positional data with the coordinate system of the reference image. In the some examples, in the case of a kidney, the scope can be navigated to one or more of the infundibulum in the kidney using an imaging device on the scope. Positional data from EM sensors about the position of the infundibulum can be aligned with the positions of the infundibulum in the reference image to achieve registration. Other methods for image-based registration (i.e., correlating positions recognized in images captured with an imaging device on the scope with positions in the reference image) are also possible. As another registration example, the reference image or another image of the anatomy (e.g., a CT image) can be captured while the scope is positioned within the anatomy such that the scope itself appears in the image. The position of the scope as determined via the position data (e.g., from the EM data) and the position of the scope in the image can be used to align the coordinate frames of both the position data and the reference image. Other registration methods are also possible.

In some implementations, after registration, the position of the instrument can be displayed on the reference image as the instrument moves within the interior region. In some embodiments, the visual indicia are displayed as breadcrumbs (e.g. data points, traces, or any other suitable visual indicator) indicative of historical (e.g., previous) positions of the instrument. The displayed visual indicia may aid the physician in verifying that all calyces of the kidney have been navigated to with the instrument. In some implementations, as the physician navigates the instrument through the kidney, the displayed visual indicia form a map representative of the anatomy of the kidney. A physician may use this map to revisit a previously visited location within the kidney and/or determine whether the instrument is currently positioned within a new (i.e., not previously visited) calyx.

In some implementations, the visual indicia can comprise a three-dimensional mesh structure representative of the anatomy of the kidney. In this way, as the physician navigates the instrument through the kidney, the visual indicia can build a three-dimensional model of the previously visited anatomy of the kidney. In some implementations, the mesh is derived from the positional information received from the EM sensor, as well as, in some cases, image data received from an imaging device on the instrument.

The systems and techniques for mapping and/or navigation described herein may allow for tagging of locations and/or features of interest while navigating the instrument through the anatomy. For example, as a physician is navigating she can "tag" points of interest such as "calyx 1," "upper pole," "large stone," etc. In some implementations, when a point is tagged, an image (e.g., taken with the imaging device on the instrument) is automatically saved along with the position (e.g., as may be determined by the EM sensor) at which the location was taken. The tagged point can be displayed on the reference image and the system may allow the user to add supplemental data, such as a label or written description. In some implementations, during subsequent navigation (either during the same or a subsequent procedure), the systems and techniques for mapping and/or navigation described herein can determine when the instrument is positioned near a previously tagged point and bring up the saved image for reference or allow the user to recall the tagged point based on a user selection.

In addition to being able to create three-dimensional maps of the kidney, the systems and techniques for mapping and/or navigation can be used to correlate recorded images or videos (e.g., taken with the imaging device on the instrument) with their anatomical location and orientation inside the kidney. This may allow physicians to understand which calyx they are viewing and/or allow them to recall images from a specific location.

Superimposing visual indicia derived from the positional information received from the EM sensor onto the reference image of the anatomy may allow a physician to contextualize the data. This may allow the physician to visualize the anatomy and instrument's position within the anatomy. This may further allow the physician to identify any regions of the kidney she may have missed. Tagging can further enhance a physician's ability to understand the visual indicia, and recalling images of tagged locations can allow the physician to perform a visual confirmation that the tagged location is the same as what was previously observed. These systems and techniques can reduce procedural time, radiation exposure, and/or error rate of missed calyces.

Conventional ureteroscopy today relies on multiple fluoroscopy images to confirm that the ureteroscope has been to each calyx. Normally the kidney is not filled with contrast during the procedure, meaning that there are very few anatomical landmarks to confirm that the correct calyx has been accessed.

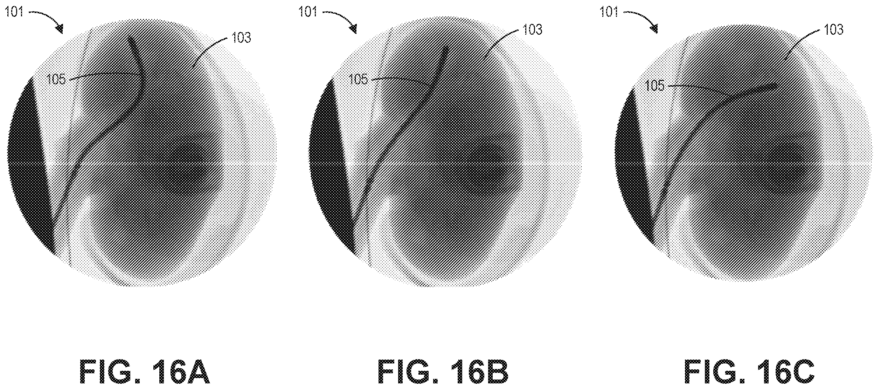

For example, FIGs. 16A-16C illustrate three example fluoroscopic images taken during a ureteroscopic procedure. FIG. 16A is a first example fluoroscopic image 101a illustrating an instrument navigating within a kidney 103. As shown in the image 101a, the outline of the kidney 103 is visible, but the specific internal structure of the kidney 103 is not readily apparent. An instrument 105 navigating within the kidney 103 is visible in a first position. The first position can be a first calyx of the kidney 103. The physician can take the image 101a to verify that the instrument 105 is positioned within the first calyx. FIG. 16B is a second example fluoroscopic image 101b illustrating the instrument 105 in a second position within the kidney 103. The second position can be a second calyx of the kidney 103. The physician can take the image 101b to verify that the instrument 105 is positioned within the second calyx. FIG. 16C is a third example fluoroscopic image 101c illustrating the instrument 105 in a third position within the kidney 103. The third position can be a third calyx. The physician can take the image 101c to verify that the instrument 105 is positioned within the third calyx. As shown in FIGs. 16A-16C, during a conventional ureteroscopic procedure, the physician must take multiple fluoroscopic images at different stages of the navigation in an effort to determine the location of the instrument within the kidney 103. As noted above, this may be disadvantageous because it requires multiple exposures to radiation, is time consuming, and can be error prone.

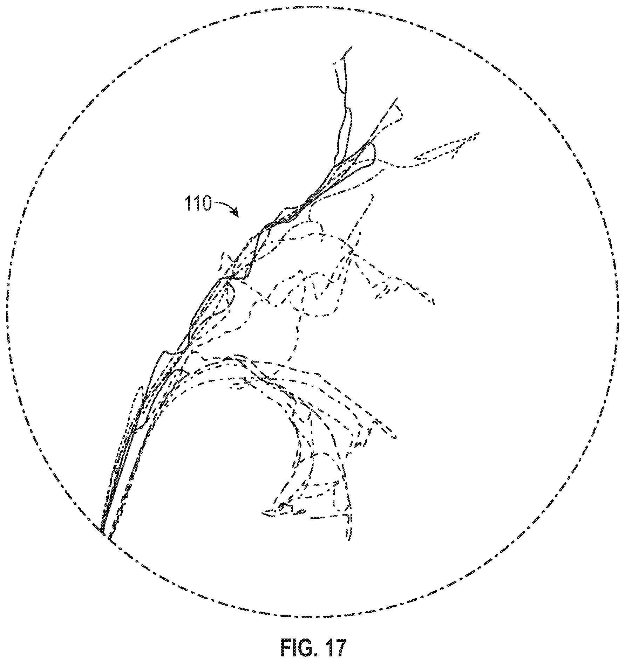

FIG. 17 illustrates an example representation of visual indicia 110 indicative of, representing, or derived from positional data received from a position sensor of an instrument navigating within a kidney. In the illustrated example, the visual indicia 110 of the positional data are displayed as a trace. In some embodiments, the trace can comprise a series of points connected by a line. The trace can be indicative of the path traveled by the instrument as it navigates within the kidney. As shown, the path can form a map that can be representative of the anatomy of the interior region. The visual indicia 110 and/or positional data can represent continuous or near continuous position tracking of the tip of the instrument as it travels through the kidney. The visual indicia 110 can create useful images or maps, especially when superimposed on a reference image of the interior region.

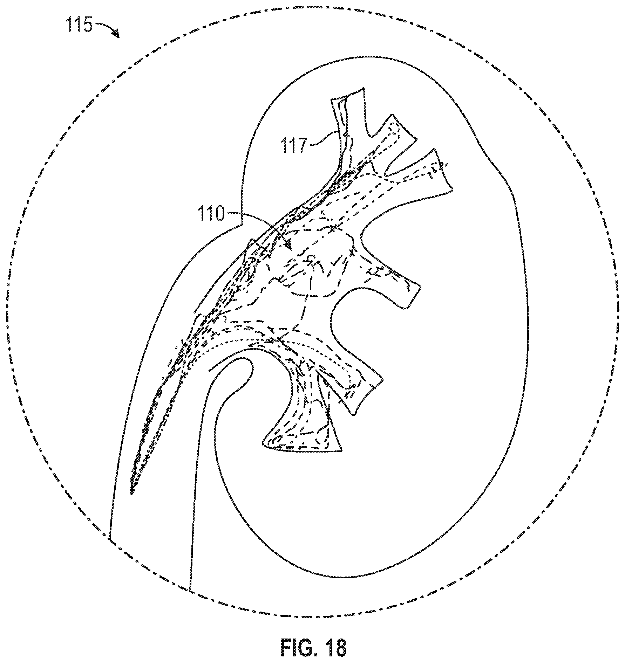

FIG. 18 illustrates the representation of the visual indicia 110 of FIG. 17 superimposed on a reference image 115 of the kidney. In this context, the visual indicia 110 provides a map of the anatomy that can be used to aid navigation. Further, the physician can tag certain locations of interest. The tagged information can also be superimposed onto the reference image. For example, in the example of FIG. 18, seven calyces of the kidney are tagged.

In some implementations, visual indicia 110 can be derived that are representative of the anatomy of the interior region. For example, as illustrated in FIG, 18, an outline 117 of the anatomy can be derived from the positional data and/or visual indicia 110 and superimposed onto the reference image. In the illustrated embodiment, the outline 117 is illustrated as a two-dimensional representation of the anatomy; however, in some implementations, the outline 117 can comprise a three-dimensional mesh representative of the anatomy. The outline 117 can thus provide a model of the interior region that can aid the physician with navigation during the procedure.

These and other features and advantages of the methods and systems for mapping and navigation of an interior region of the body will now be described in greater detail with reference to several specific example methods and systems. The described methods and systems are provided by way of example only and are intended to be illustrative of the principles of this disclosure. The disclosure should not be limited to only the described examples.

### B. Example Methods and Systems for Mapping and Navigation of an Interior Region of a Body.

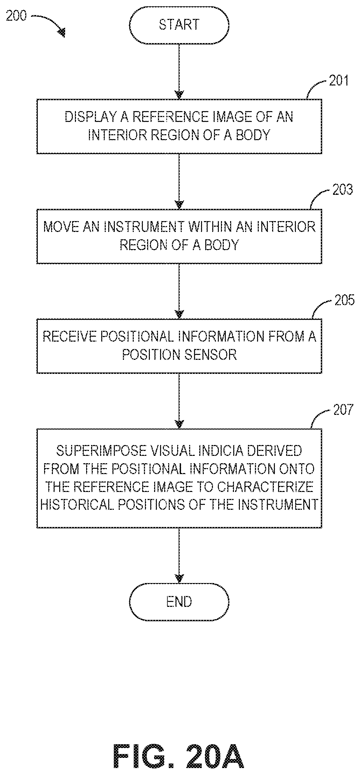

FIG. 20A is a flowchart illustrating an example method 200 for mapping an interior region of a body. The method 200 can be implemented in certain robotic systems, such as the robotic systems illustrated in FIGs. 1-15 and 22 and others. In some implementations, one or more computer devices may be configured to execute the method 200. The computer devices may be embodied by a processor (or processors) and computer-readable memory, for example, in one or more components discussed above or below. The computer-readable memory may store instructions that may be executed by the processor(s) to perform the method 200. The instructions may include one or more software modules. By way of example and not limitation, the computer devices may be in the tower 30 shown in FIG. 1, the cart shown in FIGs. 1-4, the beds shown in FIGs. 5-10, etc.

The method 200 may be executed, for example, as a medical instrument is navigated within an interior region of a body, for example, during a wide variety of medical procedures (e.g., endoscopic and/or laparoscopic procedures). The interior region can be, for example, an organ, a lumen, a luminal network, and/or a cavity, etc. In some implementations, the method 200 may be initiated when the instrument is introduced into the interior region. The method 200 may be triggered automatically (e.g., upon initialization or detection of an event) or manually (e.g., upon receipt of a user input or command).

The method 200 begins at block 201, at which a reference image of the interior region of the body is displayed. The reference image can represent or illustrate the interior region of the body. In some implementations, the reference image can be, for example, one or more of an image captured during a retrograde pyelogram procedure, a fluoroscopic image, an ultrasonic image, an image captured during a computed tomography (CT) procedure, and an image captured during a magnetic resonance imaging (MRI) procedure, or any other type of medical image.

In some implementations, the reference image can be captured intraoperatively. For example, the reference image can be captured during the medical procedure in which the method 200 is implemented. In such cases, the method 200 may include a step for capturing the reference image. In some implementations, the reference image can be captured preoperatively. For example, an image of the interior region taken prior to the procedure (e.g., during a previous procedure or previous visit to the physician) can be used as the reference image.

The reference image can be displayed to the physician performing the medical procedure. As noted previously, the reference image may provide some indication of the anatomy of the interior region. In some implementations, however, the reference image alone may not provide the physician with sufficient detail to fully comprehend the anatomy. For example, as shown in the example fluoroscopic images of FIGs. 16A-16C, fluoroscopic images may provide only a general outline of the interior region. In FIGs. 16A-16C, the general shape of the kidney 103 can be seen, while the specific interior anatomical structure and layout of the kidney (e.g., the poles, calyces, etc.) are not fully visible.

As will be discussed in greater detail below, the reference image can provide context onto which visual indicia derived from positional information received from a position sensor on the instrument can be superimposed.

The method 200 can reduce the amount of radiation exposure during the procedure or eliminate it entirely. For example, in some implementations, the patient is exposed to radiation only once during the procedure-when the reference image is captured. In contrast, during conventional ureteroscopic procedures, physicians generally take multiple fluoroscopic images (exposing the patient to multiple doses of radiation) throughout the procedure as discussed above. FIGs. 16A-16C illustrate example fluoroscopic images, all of which could be captured during a single ureteroscopic procedure. In conventional ureteroscopic procedures, the physician may rely on successive fluoroscopic images to determine that each calyx has been visited. In some implementations of the method 200, however, only a single reference image need be captured, thus limiting the amount of radiation to which the patient is exposed. Further, in some implementations of the method 200, radiation exposure during the procedure may be eliminated entirely by, for example, using a reference image that was previously captured during a previous procedure or physician visit.

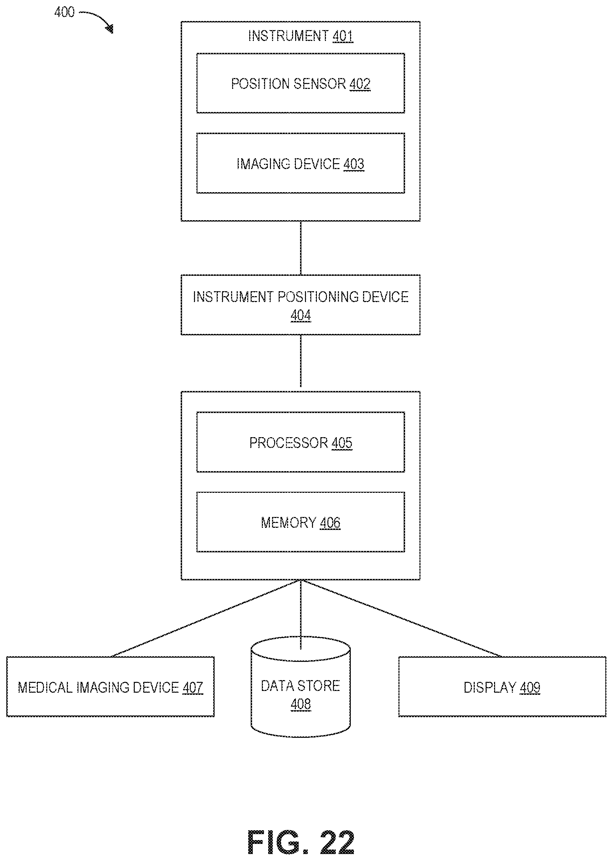

At block 203, the method 200 involves moving (e.g., navigating) an instrument within the interior region of the body. The instrument can be, for example, the endoscope 13 (FIG. 1), the ureteroscope 32 (FIG 3), the instrument 34 (FIG 4), the ureteroscope 56 (FIG. 8), the laparoscope 59 (FIG. 9), the instrument 70 (FIG. 13), or the instrument 86 (FIG. 14) described above, or any other medical instrument described herein, such as the instrument 401 (FIG. 22) described below. In some implementations, the instrument can be a robotically-enabled instrument controlled by a robotically-enabled medical system as described throughout this disclosure. In some embodiments, the instrument can be a manually controlled instrument.

The instrument can include at least one position sensor. The position sensor can be configured to provide positional information about the position (e.g., the location and/or orientation) of the instrument. The position sensor can be, for example, an EM sensor as described above. The EM sensor can be configured to provide positional information about the position (e.g., location and/or orientation) of the EM sensor within an EM field generated by an EM field generator. In some implementations, the position sensor can be an EM field generator positioned on the instrument. The position of the EM field generator can be determined relative to a plurality of EM sensors positioned external to the patient to determine the position of the instrument. In some implementations, other types of position sensors can be used. For example, the position sensor can be a shape-sensing fiber (e.g., a fiber optic shape sensor), an impedance tracker, an accelerometer, a gyroscope, an ultrasonic sensor, or any other type of sensor for determining a position of the instrument.

One or more of the position sensors may be positioned on the instrument. For example, one or more of the position sensors may be positioned on a distal tip of the instrument. In some implementations, one or more of the position sensors are not positioned on the instrument. For example, the position sensor can be a torque sensor on the proximal end of the instrument or on a motor pack of a robotic arm to which the instrument is attached. Other examples of position sensors may include movement data commanded by robotically-enabled medical system, which can be used to model an estimated pose and position of the instrument, and/or or vision data received from an imaging device on the image, which can be analyzed to determine the movement and position of the instrument.

In some implementations of the method 200, the physician controls the movement of the instrument within the interior region by, for example, providing commands to the robotically-enabled medical system. The physician may control movement of the instrument to navigate the instrument within the interior region. For example, during a ureteroscopic kidney stone removal procedure, the physician may navigate the instrument to a stone to be removed, or as described previously, the physician may navigate the instrument through each calyx of the kidney, visually inspecting each calyx to verify that stones and stone fragments have been removed. As another example, the physician may navigate the instrument to a region of interest. For example, the physician may navigate the instrument to an area of transitional cell cancer to monitor, biopsy, treat, or excise the area. As will be described below, movement or navigation of the instrument may be aided by visual indicia derived from the positional information received from the position sensor that have been superimposed on the reference image.

At block 205, the method 200 involves receiving positional information from the at least one position sensor. As noted above, the positional information may be indicative of the position (e.g., the location and/or orientation) of the instrument. In some implementations, the positional information can comprise a three degree-of-freedom position for the instrument. For example, the positional information can comprise an x, y, and z coordinate representing the position of the instrument within a Cartesian coordinate system. Other coordinate systems (e.g., polar) may also be used. In some implementations, the positional information can comprise higher degree-of-freedom positions for the instrument. For example, the positional information can comprise a five degree-of-freedom position, which includes, for example, an x, y, and z coordinate for the location as well as an indication of the pitch and yaw angles for the instrument, indicative of the orientation of the instrument. A six degree-of-freedom position for the instrument may also include roll information for the instrument.

In some implementations, the positional information includes a plurality of positional data sets. Each positional data set can indicate a position (e.g., location and/or orientation) of the instrument during the movement of the instrument. In some implementations, the positional data sets are generated continuously as the instrument is moved through the interior region. Each positional data set can indicate the position of the instrument at a particular point in time. As such, the positional data sets may comprise a log of historical positions for the instrument.

At block 207, the method 200 involves superimposing visual indicia derived from the positional information onto the reference image to characterize historical positions of the instrument. For example, block 207 can involve superimposing visual indicia derived from at least a subset of the positional data sets onto the reference image to characterize historical positions of the instrument during the movement within the interior region of the body. The superimposed visual indicia may form a map, which can aid the physician in navigating the interior region. The visual indicia can represent a path traveled by the instrument as it navigates the interior region. The visual indicia can be displayed to the physician, via a user display, superimposed on top of the reference image. The reference image thus provides a context in which to understand and visualize the displayed visual indicia.

In some implementations, the visual indicia can comprise points (e.g., breadcrumbs) that correspond to at least a subset of the positional data sets. The points can be plotted onto the reference image to display the historical positions of the instrument. The points can be represented by a wide variety of visual indicia. For example, the visual indicia can be represented as any suitable shape or marker, such as dots, dashes, X's, other shapes, etc.

Various criteria can be used to determine when to superimpose a visual indicia (i.e., the frequency at which the visual indicia are superimposed) onto the reference image. These criteria can include, for example, a distance traveled by the instrument (e.g., a distance traveled since the previous visual indicia), a direction of travel, a time elapsed since the previous superimposed visual indicia, etc. One of skill in the art will appreciate that these criteria can be varied to determine the frequency with which visual indicia are generated and superimposed onto the reference images as well as the distance between successive visual indicia.

In some implementations, a subset of the positional data sets is superimposed on the reference image at a durational frequency. The durational frequency can comprise a time between superimposed visual indicia. For example, visual indicia may be superimposed about every 0.05 seconds, i.e., at about 0.05 second time intervals. In other examples, the visual indicia may be superimposed at time intervals of about 0.1, about 0.25 seconds, or about 0.5 seconds, as well as other durations both shorter and longer than the listed examples. In some embodiments, the durational frequency may vary. For example, there need not be a fixed duration between each superimposed visual indicia.

In some implementations, the subset of the positional data sets is superimposed on the reference image at a positional frequency. The positional frequency can comprise a distance between superimposed visual indicia. For example, visual indicia can be superimposed at intervals of about 0.05 mm, about 0.1 mm, about 0.25 mm, about 0.5 mm, about 1.0 mm, or about 2.0 mm, as well as other distances both shorter and longer than the listed examples. In some embodiments, the positional frequency may vary. For example, there need not be a fixed distance between each superimposed visual indicia.

In some implementations, the visual indicia are superimposed onto the reference image substantially continuously so as to form a continuous trace representing the historical positions of the instrument (see FIGs. 17 and 18, for example). In some implementations, visual indicia that have been superimposed at a durational or positional frequency (e.g., as discrete points) can be connected to display the historical path of the movement of the instrument. For example, in some implementations, lines may be used to connect adjacent superimposed visual indicia. In another example, a curve may be fit to the superimposed visual indicia.

As the physician navigates the instrument through the interior region and the visual indicia are superimposed on the reference image, the visual indicia may begin to form a map. The map may be representative of the historical positions of the instrument. The map may also be representative of the anatomy of the interior region. For example, as the physician navigates into and out of each calyx, the paths formed by the visual indicia can be representative of the structure of each calyx. The physician can use this map to determine whether the instrument is currently being navigated into a new (i.e., a previously unvisited calyx) or to return to a previously visited calyx.

In some implementations, the visual indicia superimposed on the reference image can comprise a mesh representative of the anatomy of the interior region. For example, in some instances, tube-like structures can be fitted around the positional data sets to develop a three-dimensional mesh or model representative of the anatomy. The diameters or other shapes of the tube-like structures can be determined based on the movement of the instrument within the interior region. In some implementations, the diameters or other shapes of the tubes are determined based on the positional data sets and images received from an imaging device positioned on the instrument. For example, the method can estimate a diameter of the tube at a given position from an image captured at that position. From this, a three-dimensional mesh representative of the anatomy of the interior region can be developed and displayed as visual indicia superimposed onto the reference image.

In some instances, the visual indicia may vary over time. For example, visual indicia may fade over time such that more recently traveled portions are darker than previously traveled portions. As another example, visual indicia from different regions of the interior region can be represented as different colors (see, for example, FIGs. 17 and 18) in which different colors are represented by lines of different types of dashing. For example, different colors can be used to show paths through each different calyx.

In some implementations, data can be associated with the visual indicia. For example, a physician can create a note containing data associated with certain visual indicia. The user may enter the data via a command console and the data can be viewed via the display.

The visual indicia superimposed on the reference image can be displayed to the user intraoperatively (during the procedure). The visual indicia superimposed on the reference image can be stored or saved for use during a future medical procedure.

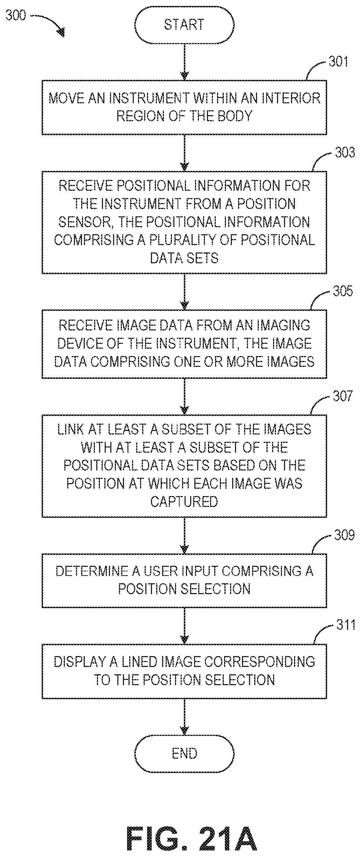

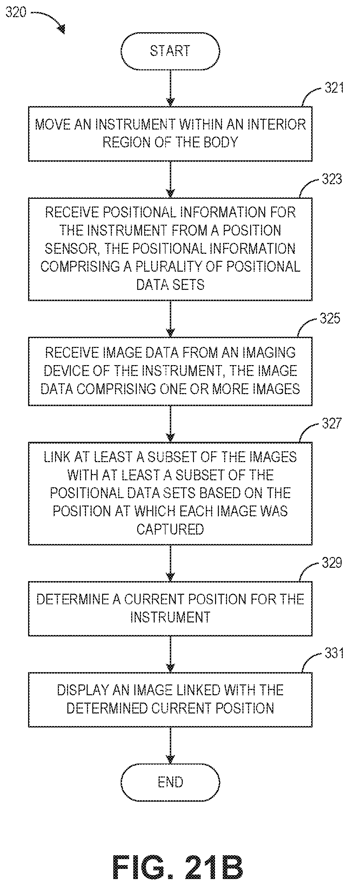

In some implementations, the method 200 can include one or more additional steps. For example, the method 200 can include one or more of the steps of the method 300 (FIG. 21A) and/or the method 320 (FIG. 21B) described below.

The method 200 may also operate to tag a location or feature of interest. For example, the method 200 can include receiving and displaying a current image from an imaging device positioned on the instrument. A physician viewing the image, can determine that the image contains a location or feature of interest. For example, the physician may determine that the image contains an entrance to a calyx, a pole of the kidney, an area of transitional cell cancer, a kidney stone, a stone fragment, etc. The physician may tag this location. The physician may input data related to the tagged location. The tagged location and/or the input data may be superimposed on the reference image (see, for example, FIG. 18 which shows seven calyces of the kidney that have been tagged).

In some implementations, the method 200 can operate to automatically detect and tag features of interest. For example, images received from the imaging device on the instrument can be processed and analyzed to determine whether they contain any locations or features of instrument. For example, an automated process may detect stones, stone fragments, or entrances to calyces within an image and tag these locations automatically. As before, the tagged locations and/or associated data can be superimposed onto the reference image.

In some implementations, the method 200 can operate to adjust a position determined by the position sensor to account for a physiological movement, and superimposing the adjusted position onto the reference image. For example, movement sensors can be positioned on the patient. The movement sensors can detect movement of the patient (e.g., breathing) and adjust the position determined by the position sensor to account for the movement of the patient.

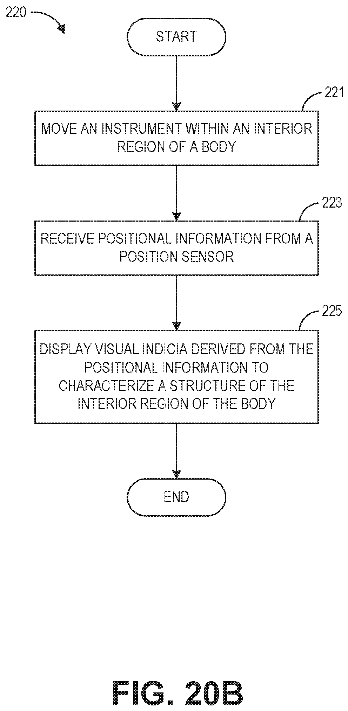

FIG. 20B is a flowchart illustrating another example method 220 for mapping an interior region of a body. The method 220 can be implemented in certain robotic systems, such as the robotic systems illustrated in FIGs. 1-15 and 22 and others. In some implementations, one or more computer devices may be configured to execute the method 220. The computer devices may be embodied by a processor (or processors) and computer-readable memory, for example, in one or more components discussed above or below. The computer-readable memory may store instructions that may be executed by the processor(s) to perform the method 220. The instructions may include one or more software modules. By way of example and not limitation, the computer devices may be in the tower 30 shown in FIG. 1, the cart shown in FIGs. 1-4, the beds shown in FIGs. 5-10, etc.

The method 220 may be executed, for example, as a medical instrument is navigated within an interior region of a body, for example, during a wide variety of medical procedures (e.g., endoscopic and/or laparoscopic procedures). The interior region can be, for example, an organ, a lumen, a luminal network, and/or a cavity, etc. In some implementations, the method 220 may be initiated when the instrument is introduced into the interior region. The method 220 may be triggered automatically (e.g., upon initialization or detection of an event) or manually (e.g., upon receipt of a user input or command).

The method 220 begins at block 221, at which an instrument is moved within an interior region of the body. At block 223, the method 220 involves receiving positional information for the instrument from a position sensor. The blocks 221, 223 can be similar to the blocks 203, 205 of the method 200, which have been previously described. For brevity, the features of blocks 221, 223 are not described again here.

At block 225, the method 220 involves displaying visual indicia derived from the positional information to characterize a structure of the interior region of the body. In many respects, the block 225 is similar to the block 207 of method 200. However, in contrast to the block 207 of the method 200, block 225 does not necessarily involve superimposing the visual indicia onto a reference image. Indeed, in some implementations, the method 220 may not involve the use of a reference image at all. Instead, block 225 can involve displaying visual indicia derived from the positional information directly to the user (i.e., without reference to any reference image). An example of such visual indicia is shown in FIG. 17, which illustrates positional information represented as visual indicia 110. As shown in FIG. 17, although no reference image is shown, in some instances, the visual indicia 110 alone may be sufficient to characterize a structure of the interior region of the body. For example, by following the traces of the visual indicia 110 in FIG. 17, the physician may gain an understanding of the general anatomical structure of the previously navigated regions of the interior region. The visual indicia 110 displayed at block 225 can be any type of visual indicia described herein, including visual indicia displayed at discrete points, visual indicia connected by lines or fitted with lines, meshes representing anatomy, etc.

The method 220 may advantageously build a model or map of the interior region of the body as the instrument navigates through the interior region. In some instances, the model or map can be used by the physician to return to a previously visited location or to determine when the instrument is navigating into a new portion of the interior region. In some instances, the more the instrument is navigated within the interior region, the clearer the map or model becomes.

In some implementations, the method 220 can include one or more additional blocks. For example, the method 220 can include one or more of the blocks of the method 300 (FIG. 21A) and/or the method 320 (FIG. 21B) described below.

FIG. 21A is a flowchart illustrating an example method 300 for navigation of an interior region of a body. The method 300 can be implemented in certain robotic systems, such as the robotic systems illustrated in FIGs. 1-15 and 22 and others. In some implementations, one or more computer devices may be configured to execute the method 300. The computer devices may be embodied by a processor (or processors) and computer-readable memory, for example, in one or more components discussed above or below. The computer-readable memory may store instructions that may be executed by the processor(s) to perform the method 300. The instructions may include one or more software modules. By way of example and not limitation, the computer devices may be in the tower 30 shown in FIG. 1, the cart shown in FIGs. 1-4, the beds shown in FIGs. 5-10, etc.

The method 300 may be executed, for example, as a medical instrument is navigated within an interior region of a body, for example, during a wide variety of medical procedures (e.g., endoscopic and/or laparoscopic procedures). The interior region can be, for example, an organ, a lumen, a luminal network, and/or a cavity, etc. In some implementations, the method 300 may be initiated when the instrument is introduced into the interior region. The method 300 may be triggered automatically (e.g., upon initialization or detection of an event) or manually (e.g., upon receipt of a user input or command).

The method 300 begins at block 301, at which an instrument is moved within an interior region of a body. The instrument can be, for example, the endoscope 13 (FIG. 1), the ureteroscope 32 (FIG 3), the instrument 34 (FIG 4), the ureteroscope 56 (FIG. 8), the laparoscope 59 (FIG. 9), the instrument 70 (FIG. 13), or the instrument 86 (FIG. 14) described above, or any other medical instrument described herein, such as the instrument 401 (FIG. 22) described below. In some implementations, the instrument can be a robotically-enabled instrument controlled by a robotically-enabled medical system as described throughout this disclosure.

The instrument can include a position sensor, such as an EM sensor, an EM field generator, a shape-sensing fiber (e.g., a fiber optic shape sensor), an impedance tracker, an accelerometer, a gyroscope, an ultrasonic sensor, or any other type of sensor for determining a position of the instrument.

In some implementations of the method 300, the physician controls the movement of the instrument within the interior region by, for example, providing commands to the robotically-enabled medical system as previously described. The physician may control movement of the instrument to navigate to the instrument to desired locations within the interior region as previously described.

At block 303, the method 300 involves receiving positional information from at least one position sensor of the instrument during movement the instrument. The positional information may be indicative of the position (e.g., the location and/or orientation) of the instrument as previously described. In some implementations, the positional information can comprise a three degree-of-freedom position for the instrument. In some implementations, the positional information can comprise higher degree-of-freedom positions for the instrument, such as five degree-of-freedom positions or six degree-of-freedom positions.

In some implementations, the positional information includes a plurality of positional data sets. Each positional data set can indicate a position (e.g., location and/or orientation) of the instrument during the movement of the instrument. In some implementations, the positional data sets are generated continuously as the instrument is moved through the interior region. Each positional data set can indicate the position of the instrument at a particular point in time. As such, the positional data sets may comprise a log of historical positions for the instrument.

At block 305, the method 300 involves receiving image data from an imaging device of the instrument within the interior region during the movement of the instrument. The imaging device may be any photosensitive substrate or structure configured to convert energy representing received light into electric signals, for example, a charge-coupled device (CCD) or complementary metal-oxide semiconductor (CMOS) image sensor. In some examples, the imaging device can include one or more optical fibers. For example, the imaging device may be a fiber optic bundle configured to transmit light representing an image from the distal end of the instrument to an image sensor. Images captured by the imaging device can be transmitted as individual frames or as series of successive frames (e.g., a video) to a computer system for storage or display.

The image data can include one or more images or videos captured by the imaging device at one or more locations within the interior region. In some implementations, the imaging device is configured to capture image data automatically. For example, the imaging device can be configured to capture images at a durational frequency. The durational frequency can comprise a time between captured images. For example, images can be captured at intervals of about 0.05 seconds, about 0.1 seconds, about 0.25 seconds, or about 0.5 seconds, as well as other durations both shorter and longer than the listed examples. In some embodiments, the durational frequency may vary. For example, there need not be a fixed duration between each captured image. The imaging device can be configured to capture images at a positional frequency. For example, the positional frequency can comprise a distance moved by the instrument between captured images. For example, images can be captured at intervals of about 0.05 mm, about 0.1 mm, about 0.25 mm, about 0.5 mm, about 1.0 mm, or about 2.0 mm, as well as other distances both shorter and longer than the listed examples. In some embodiments, the positional frequency may vary. For example, there need not be a fixed distance between each captured image. In some embodiments, images are captured substantially continuously.

In some implementations, images are captured upon receipt of a user command. For example, a physician can choose when to capture an image.

At block 307, the method 300 involves linking at least a subset of the one or more images with at least a subset of the positional data sets based on the position, as determined by the position sensor, at which each image was captured. For example, for at least a subset of the one or more images, the positional data received at the position at which the image was taken can be linked to (for example, saved with) the image. The images can be linked with a three degree-of-freedom position (e.g., a point in space). The images can also be linked with orientation information, for example, by linking the images with higher order degree-of-freedom positions, such as the five or six degree-of-freedom positions previously described. At block 307, the method 300 may create a database of images that are associated or linked with the positions at which the images were captured. As discussed below, images can be recalled from the database based on their associated or linked position for display to the physician.

In some implementations, all captured images are linked with their associated positional data sets. In some implementations, only a subset of the one or more images are linked with their associated positional data sets. The subset can be selected at a durational frequency (for example, a durational frequency of about 0.1 seconds, about 0.25 seconds, about 0.5 seconds, or about 1.0 seconds) or at a positional frequency (for example, a positional frequency of about 0.1 mm, about 0.25 mm, about 0.5 mm, or about 1.0 mm).

At block 309, the method 300 involves determining a user input comprising a position selection. In some implementations, determining the user command comprises receiving the user command. For example, a user can select a position within the interior region. In some implementations, the user selects a position from among the displayed visual indicia. In some implementations, the user selects a position relative to the reference image.

At block 311, the method 300 involves displaying a linked image of the linked images corresponding to the position selection. For example, an image linked to the selected position can be retrieved from the database previously described. In some implementations, the method 300 retrieves and displays the linked images that most closely corresponds to the selected position if there is no linked image corresponding to the selected position.

Accordingly, the method 300 may allow a user to view images corresponding to particular locations within the interior region. This may facilitate navigation within the interior region. This may aid the physician in finding and returning to a particular location or feature of interest. In certain implementations, the physician may compare a current image taken with the imaging device on the instrument to one or more previously taken images that are linked to positions within the interior region to facilitate navigation of the instrument.

FIG. 21B is a flowchart illustrating another example method 320 for navigation of an interior region of a body. The method 320 can be implemented in certain robotic systems, such as the robotic systems illustrated in FIGs. 1-15 and 22 and others. In some implementations, one or more computer devices may be configured to execute the method 320. The computer devices may be embodied by a processor (or processors) and computer-readable memory, for example, in one or more components discussed above or below. The computer-readable memory may store instructions that may be executed by the processor(s) to perform the method 320. The instructions may include one or more software modules. By way of example and not limitation, the computer devices may be in the tower 30 shown in FIG. 1, the cart shown in FIGs. 1-4, the beds shown in FIGs. 5-10, etc.

The method 320 may be executed, for example, as a medical instrument is navigated within an interior region of a body, for example, during a wide variety of medical procedures (e.g., endoscopic and/or laparoscopic procedures). The interior region can be, for example, an organ, a lumen, a luminal network, and/or a cavity, etc. In some implementations, the method 320 may be initiated when the instrument is introduced into the interior region. The method 320 may be triggered automatically (e.g., upon initialization or detection of an event) or manually (e.g., upon receipt of a user input or command).

The method 320 begins at block 321, at which an instrument is moved within an interior region of a body. The instrument can be, for example, the endoscope 13 (FIG. 1), the ureteroscope 32 (FIG 3), the instrument 34 (FIG 4), the ureteroscope 56 (FIG. 8), the laparoscope 59 (FIG. 9), the instrument 70 (FIG. 13), or the instrument 86 (FIG. 14) described above, or any other medical instrument described herein, such as the instrument 401 (FIG. 22) described below. In some implementations, the instrument can be a robotically-enabled instrument controlled by a robotically-enabled medical system as described throughout this disclosure.

The instrument can include at least one position sensor as described throughout this disclosure. In some implementations of the method 300, the physician controls the movement of the instrument within the interior region by, for example, providing commands to the robotically-enabled medical system. The physician may control movement of the instrument to navigate the instrument to desired locations within the interior region.

At block 323, the method 320 involves receiving positional information from at least one position sensor of the instrument during movement the instrument. The positional information may be indicative of the position (e.g., the location and/or orientation) of the instrument as previously described. In some implementations, the positional information can comprise a three degree-of-freedom position for the instrument. In some implementations, the positional information can comprise higher degree-of-freedom positions for the instrument, such as five degree-of-freedom positions or six degree-of-freedom positions.

In some implementations, the positional information includes a plurality of positional data sets. Each positional data set can indicate a position (e.g., location and/or orientation) of the instrument during the movement of the instrument. In some implementations, the positional data sets are generated continuously as the instrument is moved through the interior region. Each positional data set can indicate the position of the instrument at a particular point in time. As such, the positional data sets may comprise a log of historical positions for the instrument.

At block 325, the method 320 involves receiving image data from an imaging device of the instrument within the interior region during the movement of the instrument. The image data can include one or more still images of videos of the interior region. Block 325 can be similar to the block 305 of the method 300. For the sake of brevity, the features of block 325 will not be described again here.

At block 327, the method 320 involves linking at least a subset of the one or more images with at least a subset of the positional data sets based on the position, as determined by the position sensor, at which each image was captured. For example, for at least a subset of the one or more images, the positional data received at the position at which the image was taken can be linked to (for example, saved with) the image. Block 327 can be similar to the block 307 of the method 300. For the sake of brevity, the features of block 327 will not be described again here.

at block 329, the method 320 involves determining, with the at least one position sensor, a current position of the instrument, the current position corresponding a current positional data set of the plurality of positional data sets. For example, the method 320 can, at block 329, determine the current position of the instrument within the interior region.

At block 331, the method 320 involves displaying a linked image of the linked images corresponding to determined current position. Thus, the method 320 involves determining whether any linked image is available for the currently determined position of the instrument, and displaying the linked image to the physician is available. This may advantageously permit the physician to verify the location of the instrument by comparing the linked image to a current image taken with the imaging device. If the images match, the physician may conclude the instrument is in a location that it has previously been navigated to.

The method 320 may also allow the physician to compare images taken over time. For example, the physician may navigate the instrument to a location of instrument (e.g., an area of transitional cell cancer) during a subsequent procedure. The method 320 may retrieve and display an image of the location of interest taken during a previous procedure. The physician can compare the linked image and the current image to determine changes in the location of interest.

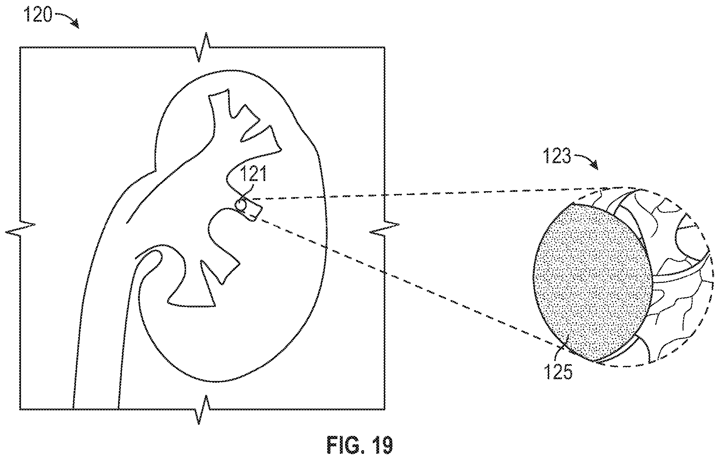

FIG. 19 illustrates an example of a display of a linked image. As shown in FIG. 19, a display 120 may provide a representation of the anatomy. In some embodiments, the display 120 shows an image of the anatomy (e g., a fluoroscopic or other medical image). In some embodiments, the display 120 shows a representation of the anatomy, such as a computer model. A user can select a location 121 as described above with reference to FIG. 21A. In another embodiment, the location 121 may be representative of the current location of an instrument within the anatomy, for example, as described above with reference to FIG. 21B. If a linked image 123 is available for the selected location, the linked image 123 can also be displayed to the user. In the illustrated embodiment, the linked image 123 shows a kidney stone 125.

FIG. 22 is a block diagram illustrating certain components of an embodiment of a system 400 for mapping and/or navigation of an interior region of a body. In the illustrated embodiment, the system 400 includes an instrument 401. The instrument 401 can include an elongate body configured for navigation of the interior region of the body. In some implementations, the elongate body can include a working channel through which one or more tools can be inserted. The elongate body can be articulable. That is, in some implementations, the shape or pose of the elongate body can be controlled. For example, the elongate body may include one or more pull wires or tendons operable to adjust the shape or pose of the elongate body.

The instrument 401 also includes a position sensor 402. The position sensor can be configured to provide positional information about the position (e.g., the location and/or orientation) of the instrument 401. The position sensor 402 can be, for example, an EM sensor as described above. The EM sensor can be configured to provide positional information about the position (e.g., location and/or orientation) of the EM sensor within an EM field generated by an EM field generator. In some implementations, the position sensor can be an EM field generator positioned on the instrument. The position of the EM field generator can be determined relative to a plurality of EM sensors positioned external to the patient to determine the position of the instrument. In some implementations, other types of position sensors can be used. For example, the position sensor 402 can be a shape-sensing fiber (e.g., a fiber optic shape sensor), an impedance tracker, an accelerometer, a gyroscope, an ultrasonic sensor, or any other type of sensor for determining a position of the instrument 401.

As illustrated, the position sensor 402 can be positioned on the instrument 401. For example, the position sensor 402 can be positioned on the distal tip of the elongate body. In some embodiments, the position sensor 402 is not positioned on the instrument 401, such as torque sensors on the proximal end of the instrument 401 or on a motor pack of a robotic arm to which the instrument is attached. Other examples of position sensors may include movement data commanded by robotically-enabled medical system, which can be used to model an estimated pose and position of the instrument, and/or or vision data received from an imaging device on the image, which can be analyzed to determine the movement and position of the instrument.

The instrument 401 also includes an imaging device 403. The imaging device 403 may be any photosensitive substrate or structure configured to convert energy representing received light into electric signals, for example, a charge-coupled device (CCD) or complementary metal-oxide semiconductor (CMOS) image sensor. In some examples, the imaging device 403 can include one or more optical fibers. For example, the imaging device 403 may be a fiber optic bundle configured to transmit light representing an image from the distal end of the instrument to an image sensor. Images captured by the imaging device 403 can comprise still images and/or video.

In the illustrated embodiment of the system 400, the instrument 401 is attached to an instrument positioning device 404. The instrument positioning device 404 can be configured to manipulate the instrument 401. For example, the instrument positioning device 404 can be configured to insert or retract the instrument 401 into the interior region of the body and/or to control the articulation (i.e., adjust the shape or pose of the instrument 401). For example, in some embodiments, the instrument positioning device 404 comprises one or more robotic arms configured to move to advance or retract the instrument 401. The instrument positioning device 404 can also include an instrument device manipulator as described above. The instrument device manipulator can be configured to actuate the pull wires or tendons of the instrument 401 to control the articulation of the instrument 401.

The system 400 includes a processor 405 and memory 406. The memory 406 may include instructions that configured the processor 405 to execute various methods or processes. For example, the memory 406 may include instructions that cause the processor 405 to execute the method 200 (FIG. 20A), the method 220 (FIG. 20B), the method 300 (FIG. 21A), and/or the 320 (FIG. 22B) described above.

As illustrated, the system 400 also includes a medical imaging device 407. The medical imaging device 407 may be configured to capture a reference image of the interior region of the body. The medical imaging device 407 may be any type of medical imager, including for example, an X-ray machine, an ultrasound, a CT scanner, or an MRI machine. The medical imaging device 407 may be connected to the processor 405 and memory 406 so as to provide the reference image for use as described above.

The system 400 also includes a data store 408. That data store 408 can be a memory, such as a hard disk drive, solid state drive, flash drive, etc., for storing information. In some implementations, the data store 408 can be configured to store positional information received from the position sensor 402 and image data received from the imaging device403. In some implementations, the data store 408 stores the image data in manner that is linked to the positional data, such that the position at which each image was captured is linked to the image. The data store 408 can also be configured store the visual indicia that are generated when the processor 405 executes the methods 200 and 220.

The system 400 also includes a display 409. The display 409 can comprise, for example, an electronic monitor (e.g., a liquid crystal display (LCD) display, a LED display, or a touch-sensitive display), a virtual reality viewing device (e.g., goggles or glasses), and/or other display devices.

The display 409 can be configured to display various information to a physician during a procedure. For example, the display 409 can display the reference image, as well as the visual indicia superimposed thereon. As another example, the display 409 can be configured to display one or more tagged images related to a user selection of position.

### 3. Implementing Systems and Terminology.

Implementations disclosed herein provide systems, methods and apparatuses for mapping and/or navigation of an interior region of a body with a robotically-enabled medical instrument.

It should be noted that the terms "couple," "coupling," "coupled" or other variations of the word couple as used herein may indicate either an indirect connection or a direct connection. For example, if a first component is "coupled" to a second component, the first component may be either indirectly connected to the second component via another component or directly connected to the second component.

The mapping and navigation functions described herein may be stored as one or more instructions on a processor-readable or computer-readable medium. The term "computer-readable medium" refers to any available medium that can be accessed by a computer or processor. By way of example, and not limitation, such a medium may comprise random access memory (RAM), read-only memory (ROM), electrically erasable programmable read-only memory (EEPROM), flash memory, compact disc read-only memory (CD-ROM) or other optical disk storage, magnetic disk storage or other magnetic storage devices, or any other medium that can be used to store desired program code in the form of instructions or data structures and that can be accessed by a computer. It should be noted that a computer-readable medium may be tangible and non-transitory. As used herein, the term "code" may refer to software, instructions, code or data that is/are executable by a computing device or processor.

The methods disclosed herein comprise one or more steps or actions for achieving the described method. The method steps and/or actions may be interchanged with one another without departing from the scope of the claims. In other words, unless a specific order of steps or actions is required for proper operation of the method that is being described, the order and/or use of specific steps and/or actions may be modified without departing from the scope of the claims.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

The previous description of the disclosed implementations is provided to enable any person skilled in the art to make or use the present invention. Various modifications to these implementations will be readily apparent to those skilled in the art, and the generic principles defined herein may be applied to other implementations without departing from the scope of the invention. For example, it will be appreciated that one of ordinary skill in the art will be able to employ a number corresponding alternative and equivalent structural details, such as equivalent ways of fastening, mounting, coupling, or engaging tool components, equivalent mechanisms for producing particular actuation motions, and equivalent mechanisms for delivering electrical energy. Thus, the present invention is not intended to be limited to the implementations shown herein but is to be accorded the widest scope consistent with the principles and novel features disclosed herein.

## Claims

1. A non-transitory computer readable storage medium having stored thereon instructions that, when executed by a processor of a robotic surgical system (400) causes said robotic surgical system (400) to at least:
move (301) an instrument (401) of the robotic surgical system within an interior region of a body;
receive (303) positional information from at least one position sensor (402) of the instrument (401) during the movement of the instrument (401), the positional information comprising a plurality of positional data sets, each positional data set indicative of a position of the instrument (401);
display a reference image (115) of the interior region of the body on a display (409) of the robotic surgical system (400);
superimpose visual indicia (110) derived from at least a subset of the positional data sets on the reference image (115) to characterize historical positions of the instrument during the movement of the instrument (401) within the interior region of the body;
receive (305) image data from an imaging device (403) of the robotic surgical system (400) positioned on the instrument (401), the image data comprising a plurality of images captured by the imaging device during the movement of the instrument (401);
for at least a subset of the plurality of images, link (307) each image of the subset with the positional data set indicative of a position at which the image was captured; and
receive (309) a user input of a position selection and display (311) a linked image of the subset of the plurality of images corresponding to the user input.

2. The non-transitory computer readable storage medium of Claim 1, wherein the reference image comprises an image captured during a retrograde pyelogram procedure, a fluoroscopic or an ultrasonic image, or wherein the reference image is captured during a computed tomography (CT) or magnetic resonance imaging (MRI) procedure.

3. The non-transitory computer readable storage medium of Claim 1, wherein the instructions, when executed, further cause the processor to capture the reference image intraoperatively.

4. The non-transitory computer readable storage medium of Claim 1, wherein the reference image is captured preoperatively.

5. The non-transitory computer readable storage medium of Claim 1, wherein the instructions, when executed, further cause the processor to:
store the linked images for use during a future procedure;
determine, with the position sensor, a current position of the instrument and display a linked image corresponding to the determined current position.

6. The non-transitory computer readable storage medium of Claim 1, wherein the image data is captured automatically, or upon receipt of a user command.

7. The non-transitory computer readable storage medium of Claim 1, wherein the instructions, when executed, further cause the processor to tag a location or feature of interest within the interior region, optionally wherein tagging comprises receiving a user input, or automatically detecting the location or feature of interest.

8. The non-transitory computer readable storage medium of Claim 7, wherein the instructions, when executed, further cause the processor to superimpose the tagged location or feature of interest on the reference image.

9. The non-transitory computer readable storage medium of Claim 1, wherein the instructions, when executed, further cause the processor to connect the visual indicia to characterize the historical path of the movement of the instrument
wherein to connect the visual inidicia comprises rendering one of: a line that connects adjacent visual indicia or a curve fit to the visual indicia.

10. The non-transitory computer readable storage medium of Claim 1, wherein the visual indicia comprise a mesh, optionally wherein the mesh is indicative of anatomy of the interior region, further optionally wherein the mesh is derived from the subset of the positional data sets and image data received from an imaging device on the instrument.

11. The non-transitory computer readable storage medium of Claim 10, wherein the mesh is a three-dimensional mesh.

12. The non-transitory computer readable storage medium of Claim 1, wherein the subset of the positional data sets is superimposed on the reference image at a durational frequency or at a positional frequency.

13. The non-transitory computer readable storage medium of Claim 1, wherein the instructions, when executed, further cause the processor to display the visual indicia intraoperatively and/or to store the visual indicia for use during a future medical procedure.

14. The non-transitory computer readable storage medium of Claim 1, wherein the instructions, when executed, further cause the processor to:
adjust a position determined by the position sensor to account for a physiological movement; and
superimpose the adjusted position on the reference image.

15. A robotic surgical system (400) comprising:
an instrument (401) having an elongate body and at least one position sensor (402) disposed on the elongate body;
a display (409);
an imaging device (403) positioned on the instrument (400);
a processor (405) in communication with the non-transitory computer readable storage medium (406) of any one of claims 1-13 and configured to execute the instructions on said readable storage medium.

## Patentansprüche

1. Nicht-flüchtiges computerlesbares Speichermedium, das Anweisungen darauf gespeichert aufweist, die, wenn sie durch einen Prozessor eines robotischen Chirurgiesystems (400) ausgeführt werden, das robotische Chirurgiesystem (400) veranlassen zum:
Bewegen (301) eines Instruments (401) des robotischen Chirurgiesystems innerhalb eines Innenbereichs eines Körpers;
Empfangen (303) von Positionsinformationen von mindestens einem Positionssensor (402) des Instruments (401) während der Bewegung des Instruments (401), die Positionsinformationen umfassend eine Vielzahl von Positionsdatensätzen, wobei jeder Positionsdatensatz eine Position des Instruments (401) angibt;
Anzeigen eines Referenzbilds (115) des Innenbereichs des Körpers auf einer Anzeige (409) des robotischen Chirurgiesystems (400);
Überlagern von visuellen Kennzeichen (110), die von mindestens einer Teilmenge der Positionsdatensätze abgeleitet sind, auf das Referenzbild (115), um historische Positionen des Instruments während der Bewegung des Instruments (401) innerhalb des Innenbereichs des Körpers zu charakterisieren;
Empfangen (305) von Bilddaten von einer Bildgebungsvorrichtung (403) des robotischen Chirurgiesystems (400), die an dem Instrument (401) positioniert ist, die Bilddaten umfassend eine Vielzahl von Bildern, die durch die Bildgebungsvorrichtung während der Bewegung des Instruments (401) aufgenommen wurden;
für mindestens eine Teilmenge der Vielzahl von Bildern, Verknüpfen (307) jedes Bildes der Teilmenge mit dem Positionsdatensatz, der eine Position angibt, an der das Bild aufgenommen wurde; und Empfangen (309) einer Benutzereingabe einer Positionsauswahl und Anzeigen (311) eines verknüpften Bildes der Teilmenge der Vielzahl von Bildern entsprechend der Benutzereingabe.

2. Nicht-flüchtiges computerlesbares Speichermedium nach Anspruch 1, wobei das Referenzbild ein Bild, das während eines retrograden Pyelogramm-Verfahrens aufgenommen wurde, ein fluoroskopisches oder ein Ultraschallbild umfasst, oder wobei das Referenzbild während eines Computertomographie(CT-) oder Magnetresonanztomographie(MRT-) Verfahrens aufgenommen wird.

3. Nicht-flüchtiges computerlesbares Speichermedium nach Anspruch 1, wobei die Anweisungen, wenn sie ausgeführt werden, den Prozessor ferner veranlassen das Referenzbild intraoperativ aufzunehmen.

4. Nicht-flüchtiges computerlesbares Speichermedium nach Anspruch 1, wobei das Referenzbild präoperativ aufgenommen wird.

5. Nicht-flüchtiges computerlesbares Speichermedium nach Anspruch 1, wobei die Anweisungen, wenn sie ausgeführt werden, den Prozessor ferner veranlassen zum:
Speichern der verknüpften Bilder zur Verwendung während eines zukünftigen Verfahrens;
Bestimmen, mit dem Positionssensor, einer aktuellen Position des Instruments und Anzeigen eines verknüpften Bildes entsprechend der bestimmten aktuellen Position.

6. Nicht-flüchtiges computerlesbares Speichermedium nach Anspruch 1, wobei die Bilddaten automatisch oder beim Empfang eines Benutzerbefehls aufgenommen werden.

7. Nicht-flüchtiges computerlesbares Speichermedium nach Anspruch 1, wobei die Anweisungen, wenn sie ausgeführt werden, den Prozessor ferner veranlassen, eine Stelle oder ein Merkmal von Interesse innerhalb des Innenbereichs zu markieren, optional wobei das Markieren das Empfangen einer Benutzereingabe oder ein automatisches Erkennen der Stelle oder des Merkmals von Interesse umfasst.

8. Nicht-flüchtiges computerlesbares Speichermedium nach Anspruch 7, wobei die Anweisungen, wenn sie ausgeführt werden, den Prozessor ferner veranlassen, die markierte Stelle oder das Merkmal von Interesse auf das Referenzbild zu überlagern.

9. Nicht-flüchtiges computerlesbares Speichermedium nach Anspruch 1, wobei die Anweisungen, wenn sie ausgeführt werden, den Prozessor ferner veranlassen, die visuellen Kennzeichen zu verbinden, um den historischen Pfad der Bewegung des Instruments zu charakterisieren
wobei das Verbinden der visuellen Kennzeichen ein Darstellen von einem umfasst von: einer Linie, die benachbarte visuelle Kennzeichen verbindet, oder einer Kurve, die an die visuellen Kennzeichen angepasst ist.

10. Nicht-flüchtiges computerlesbares Speichermedium nach Anspruch 1, wobei die visuellen Kennzeichen ein Netz umfassen, optional wobei das Netz eine Anatomie des Innenbereichs angibt, ferner optional wobei das Netz aus der Teilmenge der Positionsdatensätze und Bilddaten abgeleitet ist, die von einer Bildgebungsvorrichtung an dem Instrument empfangen werden.

11. Nicht-flüchtiges computerlesbares Speichermedium nach Anspruch 10, wobei das Netz ein dreidimensionales Netz ist.

12. Nicht-flüchtiges computerlesbares Speichermedium nach Anspruch 1, wobei die Teilmenge der Positionsdatensätze auf das Referenzbild mit einer Dauerfrequenz oder mit einer Positionsfrequenz überlagert wird.

13. Nicht-flüchtiges computerlesbares Speichermedium nach Anspruch 1, wobei die Anweisungen, wenn sie ausgeführt werden, den Prozessor ferner veranlassen, die visuellen Kennzeichen intraoperativ anzuzeigen und/oder die visuellen Kennzeichen zur Verwendung während eines zukünftigen medizinischen Verfahrens zu speichern.

14. Nicht-flüchtiges computerlesbares Speichermedium nach Anspruch 1, wobei die Anweisungen, wenn sie ausgeführt werden, den Prozessor ferner veranlassen zum:
Anpassen einer Position, die durch den Positionssensor bestimmt wird, um eine physiologische Bewegung zu berücksichtigen; und
Überlagern der angepassten Position auf das Referenzbild.

15. Robotisches Chirurgiesystem (400), umfassend:
ein Instrument (401), das einen länglichen Körper und mindestens einem Positionssensor (402), der an dem länglichen Körper angeordnet ist, aufweist;
eine Anzeige (409);
eine Bildgebungsvorrichtung (403), die an dem Instrument (400) positioniert ist;
einen Prozessor (405) in Kommunikation mit dem nicht-flüchtigen computerlesbaren Speichermedium (406) nach einem der Ansprüche 1 bis 13 und konfiguriert ist, um die Anweisungen auf dem lesbaren Speichermedium auszuführen.

## Revendications

1. Support de stockage non transitoire lisible par ordinateur sur lequel sont stockées des instructions qui, lorsqu'elles sont exécutées par un processeur d'un système chirurgical robotique (400), amènent ledit système chirurgical robotique (400) à au moins :
déplacer (301) un instrument (401) du système chirurgical robotique dans une région intérieure d'un corps ;
recevoir (303) des informations de position provenant d'au moins un capteur de position (402) de l'instrument (401) pendant le mouvement de l'instrument (401), les informations de position comprenant une pluralité d'ensembles de données de position, chaque ensemble de données de position indiquant une position de l'instrument (401) ;
afficher une image de référence (115) de la région intérieure du corps sur un écran (409) du système chirurgical robotique (400) ;
superposer à l'image de référence (115) des indices visuels (110) dérivés d'au moins un sous-ensemble des ensembles de données de position afin de caractériser des positions historiques de l'instrument pendant le mouvement de l'instrument (401) dans la région intérieure du corps ;
recevoir (305) des données d'image à partir d'un dispositif d'imagerie (403) du système chirurgical robotique (400) positionné sur l'instrument (401), les données d'image comprenant une pluralité d'images capturées par le dispositif d'imagerie pendant le mouvement de l'instrument (401) ;
pour au moins un sous-ensemble de la pluralité d'images, lier (307) chaque image du sous-ensemble à l'ensemble de données de position indiquant une position à laquelle l'image a été capturée ; et recevoir (309) une entrée d'utilisateur d'une sélection de position et afficher (311) une image liée du sous-ensemble de la pluralité d'images correspondant à l'entrée d'utilisateur.

2. Support de stockage non transitoire lisible par ordinateur selon la revendication 1, dans lequel l'image de référence comprend une image capturée au cours d'une procédure de pyélogramme rétrograde, une image fluoroscopique ou ultrasonique, ou dans lequel l'image de référence est capturée pendant une procédure de tomographie assistée par ordinateur (CT) ou d'imagerie par résonance magnétique (IRM).

3. Support de stockage non transitoire lisible par ordinateur selon la revendication 1, dans lequel les instructions, lorsqu'elles sont exécutées, amènent en outre le processeur à capturer l'image de référence pendant l'opération.

4. Support de stockage non transitoire lisible par ordinateur selon la revendication 1, dans lequel l'image de référence est capturée avant l'opération.

5. Support de stockage non transitoire lisible par ordinateur selon la revendication 1, dans lequel les instructions, lorsqu'elles sont exécutées, amènent en outre le processeur à :
stocker les images liées pour les utiliser lors d'une procédure ultérieure ;
déterminer, avec le capteur de position, une position actuelle de l'instrument et afficher une image liée correspondant à la position actuelle déterminée.

6. Support de stockage non transitoire lisible par ordinateur selon la revendication 1, dans lequel les données d'image sont capturées automatiquement ou à la réception d'une commande de l'utilisateur.

7. Support de stockage non transitoire lisible par ordinateur selon la revendication 1, dans lequel les instructions, lorsqu'elles sont exécutées, amènent en outre le processeur à marquer un emplacement ou une caractéristique d'intérêt dans la région intérieure, éventuellement dans lequel le marquage comprend la réception d'une entrée d'utilisateur, ou la détection automatique de l'emplacement ou de la caractéristique d'intérêt.

8. Support de stockage non transitoire lisible par ordinateur selon la revendication 7, dans lequel les instructions, lorsqu'elles sont exécutées, amènent en outre le processeur à superposer l'emplacement marqué ou la caractéristique d'intérêt sur l'image de référence.

9. Support de stockage non transitoire lisible par ordinateur selon la revendication 1, dans lequel les instructions, lorsqu'elles sont exécutées, amènent en outre le processeur à connecter les indices visuels pour caractériser la trajectoire historique du mouvement de l'instrument
dans lequel la liaison des indices visuels comprend le rendu de l'une parmi : une ligne qui relie des indices visuels adjacents ou une courbe ajustée aux indices visuels.

10. Support de stockage non transitoire lisible par ordinateur selon la revendication 1, dans lequel les indices visuels comprennent un maillage, éventuellement dans lequel le maillage indique une anatomie de la région intérieure, éventuellement en outre dans lequel le maillage est dérivé du sous-ensemble des ensembles de données de position et des données d'image reçues à partir d'un dispositif d'imagerie sur l'instrument.

11. Support de stockage non transitoire lisible par ordinateur selon la revendication 10, dans lequel le maillage est un maillage tridimensionnel.

12. Support de stockage non transitoire lisible par ordinateur selon la revendication 1, dans lequel le sous-ensemble des ensembles de données de position est superposé à l'image de référence à une fréquence de durée ou à une fréquence de position.

13. Support de stockage non transitoire lisible par ordinateur selon la revendication 1, dans lequel les instructions, lorsqu'elles sont exécutées, amènent en outre le processeur à afficher les indices visuels pendant l'opération et/ou à stocker les indices visuels en vue d'une utilisation lors d'une procédure médicale ultérieure.

14. Support de stockage non transitoire lisible par ordinateur selon la revendication 1, dans lequel les instructions, lorsqu'elles sont exécutées, amènent en outre le processeur à :
ajuster une position déterminée par le capteur de position pour tenir compte d'un mouvement physiologique ; et
superposer la position ajustée à l'image de référence.

15. Système chirurgical robotique (400), comprenant :
un instrument (401) ayant un corps allongé et au moins un capteur de position (402) disposé sur le corps allongé ;
un écran (409) ;
un dispositif d'imagerie (403) positionné sur l'instrument (400) ;
un processeur (405) en communication avec le support de stockage non transitoire lisible par ordinateur (406) selon l'une quelconque des revendications 1 à 13 et configuré pour exécuter les instructions sur ledit support de stockage lisible.
